(19) Europäisches Patentamt
European Patent Office
Office européen des brevets

(11) EP 1 568 311 A1

(12) **EUROPEAN PATENT APPLICATION**

(43) Date of publication:
**31.08.2005 Bulletin 2005/35**

(21) Application number: **04007818.0**

(22) Date of filing: **31.03.2004**

(51) Int Cl.[7]: **A61B 5/00**, G01K 13/00, G01K 17/08, G06F 17/00, G06F 19/00

(84) Designated Contracting States:
**AT BE BG CH CY CZ DE DK EE ES FI FR GB GR HU IE IT LI LU MC NL PL PT RO SE SI SK TR**
Designated Extension States:
**AL LT LV MK**

(30) Priority: **27.02.2004 JP 2004055391**

(71) Applicant: **Hitachi, Ltd.**
**Tokyo 101-8010 (JP)**

(72) Inventors:
- **Cho, Ok-Oyung c/o Hitachi, Ltd.**
  **Chiyoda-ku Tokyo 100-8220 (JP)**
- **Kim, Yoon-Ok c/o Hitachi, Ltd.**
  **Chiyoda-ku Tokyo 100-8220 (JP)**
- **Uchida, Tsuyoshi c/o Hitachi, Ltd.**
  **Chiyoda-ku Tokyo 100-8220 (JP)**
- **Mitsumaki,Hiroshi c/o Hitachi, Ltd.**
  **Chiyoda-ku Tokyo 100-8220 (JP)**

(74) Representative: **Strehl Schübel-Hopf & Partner**
**Maximilianstrasse 54**
**80538 München (DE)**

(54) **Blood sugar level measuring apparatus**

(57) Blood sugar levels are measured non-invasively based on temperature measurement. Non-invasively measured blood sugar level values obtained by a temperature measurement scheme are corrected by blood oxygen saturation and blood flow volume, thereby stabilizing the measurement data.
Multiple calibration curves are obtained by cluster analysis of patient data.

FIG. 9

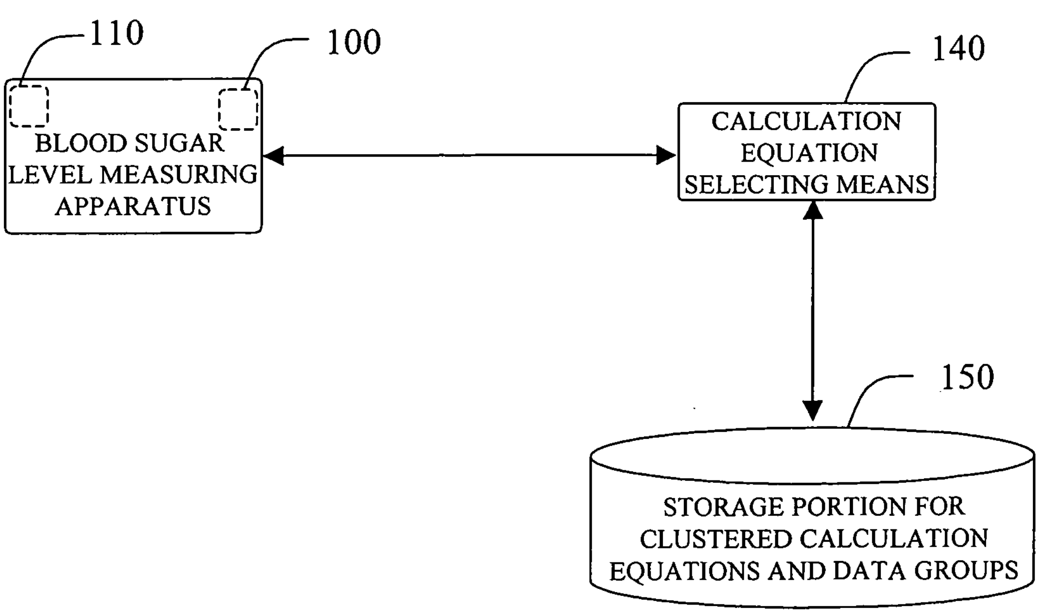


EP 1 568 311 A1

## Description

CLAIM OF PRIORITY

The present application claims priority from Japanese application JP 2004-55391 filed on February 27, 2004, the content of which is hereby incorporated by reference to this application.

BACKGROUND OF THE INVENTION

Field of the Invention

The present invention relates to a non-invasive blood sugar level measuring method and apparatus for measuring glucose concentration in a living body without blood sampling.

Background Art

Hilson *et al.* report facial and sublingual temperature changes in diabetics following intravenous glucose injection (Non-Patent Document 1). Scott *et al.* discuss the issue of diabetics and thermoregulation (Non-Patent Document 2). Based on such researches, Cho *et al.* suggests a method and apparatus for determining blood glucose concentration by temperature measurement without requiring the collection of a blood sample (Patent Documents 1 and 2).

Various other attempts have been made to determine glucose concentration without blood sampling. For example, a method has been suggested (Patent Document 3) whereby a measurement site is irradiated with near-infrared light of three wavelengths, and the intensity of transmitted light as well as the temperature of the living body is detected. Then, a representative value of the second-order differentiated values of absorbance is calculated, and the representative value is corrected in accordance with the difference between the living body temperature and a predetermined reference temperature. A blood sugar level corresponding to the thus corrected representative value is then determined. An apparatus is also provided (Patent Document 4) whereby a measurement site is heated or cooled while monitoring the living body temperature. The degree of attenuation of light based on light irradiation is measured at the moment of temperature change so that the glucose concentration responsible for the temperature-dependency of the degree of light attenuation can be measured. Further, an apparatus is reported (Patent Document 5) whereby an output ratio between reference light and the light transmitted by an irradiated sample is taken, and then a glucose concentration is calculated by a linear expression of the logarithm of the output ratio and the living body temperature. In another example, when determining body composition concentrations by measuring near-infrared spectrum of the skin, the subjects are classified according to their skin characters, and calibration curves are prepared for individual classifications (Patent Document 6).

[Non-Patent Document 1] R.M. Hilson and T.D.R. Hockaday, "Facial and sublingual temperature changes following intravenous glucose injection in diabetics," Diabete & Metabolisme, 8, pp.15-19: 1982

[Non-Patent Document 2] A.R. Scott, T. Bennett, I.A. MacDonald, "Diabetes mellitus and thermoregulation," Can. J. Physiol. Pharmacol., 65, pp. 1365-1376: 1987

[Patent Document 1] U.S. Patent No. 5,924,996

[Patent Document 2] U.S. Patent No. 5,795,305

[Patent Document 3] JP Patent Publication (Kokai) No. 2000-258343 A

[Patent Document 4] JP Patent Publication (Kokai) No. 10-33512 A (1998)

[Patent Document 5] JP Patent Publication (Kokai) No. 10-108857 A (1998)

[Patent Document 6] JP Patent Publication (Kokai) No. 2003-144421 A

SUMMARY OF THE INVENTION

Glucose (blood sugar) in blood is used for glucose oxidation reaction in cells to produce necessary energy for the maintenance of a living body. In the basal metabolism state, in particular, most of the produced energy is converted into heat energy for the maintenance of body temperature. Thus, it can be expected that there is some relationship between blood glucose concentration and body temperature. However, as is evident from the way sicknesses cause fever, the body temperature also varies due to factors other than blood glucose concentration. While methods have been proposed to determine blood glucose concentration by temperature measurement without blood sampling, they lack sufficient accuracy.

Patent Publication 6 discloses that when determining body composition concentrations or the like in the skin tissues of human or other living organisms, in which individual differences are large, the subjects are classified according to their skin characteristics and calibration formulae are prepared for individual classifications, thereby eliminating the need for preparing a calibration formula for each individual or measured portion. In this scheme, however, it is difficult to avoid the influence from components other than that as the object of determination during the measurement of body composition concentrations. Further, there is the need to measure the skin characteristics (specifically, the skin thickness) of the subjects using ultrasound tomographic measurement equipment or the like, separately from the determination of body composition concentrations or the like, resulting in complicating the measurement process.

It is the object of the invention to provide a method and apparatus for determining blood glucose concentration with high accuracy based on temperature data of a subject without blood sampling.

Blood sugar is delivered to the cells throughout the human body via the blood vessel system, particularly the capillary blood vessels. In the human body, complex metabolic pathways exist. Glucose oxidation is a reaction in which, fundamentally, blood sugar reacts with oxygen to produce water, carbon dioxide, and energy. Oxygen herein refers to the oxygen delivered to the cells via blood. The amount of oxygen supply is determined by the blood hemoglobin concentration, the hemoglobin oxygen saturation, and the volume of blood flow. On the other hand, the heat produced in the body by glucose oxidation is dissipated from the body by convection, heat radiation, conduction, and so on. On the assumption that the body temperature is determined by the balance between the amount of energy produced in the body by glucose burning, namely heat production, and heat dissipation such as mentioned above, we set up the following model:

(1) The amount of heat production and the amount of heat dissipation are considered equal.
(2) The amount of heat production is a function of the blood glucose concentration and the amount of oxygen supply.
(3) The amount of oxygen supply is determined by blood hemoglobin concentration, blood hemoglobin oxygen saturaiton, and the volume of blood flow in the capillary blood vessels.
(4) The amount of heat dissipation is mainly determined by heat convection and heat radiation.

The inventors have achieved the present invention after realizing that blood sugar levels can be accurately determined on the basis of the results of measuring the temperature of the body surface and parameters relating to blood oxygen concentration and blood flow volume, in accordance with the aforementioned model. The parameters can be measured from a part of the human body, such as the fingertip. Parameters relating to convection and radiation can be determined by carrying out thermal measurements on the fingertip. Parameters relating to blood hemoglobin concentration and blood hemoglobin oxygen saturation can be obtained by spectroscopically measuring blood hemoglobin and determining the ratio of hemoglobin bound with oxygen to hemoglobin not bound with oxygen. With regard to the parameters relating to blood hemoglobin concentration and blood hemoglobin oxygen saturation, measurement accuracy would not be significantly lowered if pre-stored constants are employed rather than taking measurements. The parameter relating to the volume of blood flow can be determined by measuring the amount of heat transfer from the skin.

In one aspect, the invention provides a blood sugar level calculation method comprising the steps of:

measuring a first temperature which is the temperature of a plate that is in contact with a body surface;
measuring a second temperature which is the temperature of the heat that is transmitted from said plate to a first member disposed adjacent to said plate;
measuring a third temperature which is the temperature of the heat radiating from said body surface;
detecting light with which said plate has been irradiated;
selecting a first calculation equation from a group of calculation equations on the basis of said first temperature, said second temperature, said third temperature, and the result of detection of said light, said group of calculation equations being obtained by classifying a plurality of data sets concerning said first temperature, said second temperature, said third temperature, and the result of detection of said light by a statistical process; and
calculating a blood sugar level by using said first temperature, said second temperature, said third temperature, said result of detection of light, and said first calculation equation.

The invention also provides a blood sugar level calculation method comprising the steps of:

measuring a first temperature which is the temperature of a plate that is in contact with a body surface;
measuring a second temperature which is the temperature of the heat that is transmitted from said plate to a columnar member disposed adjacent to said plate;
measuring a third temperature which is the temperature of the heat radiating from said body surface;
detecting light with which said plate has been irradiated;
selecting a first calculation equation from a group of calculation equations on the basis of said first temperature, said second temperature, said third temperature, and the result of detection of said light, said group of calculation equations being obtained by classifying a plurality of data sets concerning said first temperature, said second temperature, said third temperature, and the result of detection of said light by a statistical process; and calculating a blood sugar level by using said first temperature, said second temperature, said third temperature, said result of detection of light, and said first calculation equation.

The invention further provides a blood sugar level measuring system comprising:

a heat amount measuring portion for measuring a plurality of temperatures derived from a body surface and obtaining information used for calculating the amount of heat transferred by convection and the amount of heat transferred by radiation, both related to heat dissipation from said body surface;
a body surface contact portion;
an indirect temperature detector for detecting the concentration at a position spaced apart from said body surface contact portion;
a heat conducting member connecting said body surface contact portion and said indirect temperature detector;
a blood flow volume measuring portion for obtaining information relating to the volume of blood flow;
an optical measuring portion for obtaining hemoglobin concentration and hemoglobin oxygen saturation in blood;
an oxygen supply volume measuring portion for obtaining information about the amount of oxygen in blood;
a first storage portion for storing groups of measurement value sets or a calculation equation prepared for each of said groups of measurement value sets, said groups of measurement value sets being obtained by classifying a plurality of sets of measurement values obtained in advance by said heat amount measuring portion and said oxygen supply volume measuring portion by a statistical process;
a calculation equation selecting means for selecting a first calculation equation from said first storage portion based on measurement information about said plurality of temperatures and the blood oxygen amount;
a second storage portion for storing said first calculation equation;
a calculation portion for calculating a blood sugar level by using a plurality of measurement values inputted from said heat amount measuring portion and said oxygen supply volume measuring portion, and said first calculation equation stored in said second storage portion; and
a display portion for displaying the result of calculation in said calculation portion.

The invention further provides a blood sugar level measuring apparatus comprising:

an ambient temperature measuring device for measuring ambient temperature;
a body-surface contact portion to which a body surface is brought into contact;
a radiant heat detector for measuring radiant heat from said body surface;
a heat conducting member disposed in contact with said body-surface contact portion;
an indirect temperature detector disposed at a position that is adjacent to said heat conducting member and that is spaced apart from said body-surface contact portion, said indirect temperature detector measuring temperature at the position spaced apart from said body-surface contact portion;
a light source for irradiating said body-surface contact portion with light of at least two different wavelengths;
a light detector for detecting reflected light produced as said light is reflected by said body surface;
a first storage portion for storing groups of measurement value sets or a calculation equation prepared for each of said groups of measurement value sets, said groups of measurement value sets being obtained by classifying a plurality of sets of measurement values obtained in advance by said heat amount measuring portion and said oxygen supply volume measuring portion by a statistical process;
a calculation equation selecting means for selecting a first calculation equation from said first storage portion based on the outputs from said indirect temperature detector, said ambient temperature detector, said radiant heat detector, and said light detector;
a second storage portion for storing said first calculation equation;
a calculation portion for calculating a blood sugar level by using the outputs from said indirect temperature detector, said ambient temperature detector, said radiant heat detector, and said light detector, and said first calculation equation stored in said second storage portion; and
a display portion for displaying the blood sugar level outputted from said calculating portion.

Further, the invention provides a blood sugar level measuring apparatus comprising:

an ambient temperature measuring device for measuring ambient temperature;
a body-surface contact portion to which a body surface is brought into contact;
a radiant heat detector for measuring radiant heat from said body surface;
a heat conducting member disposed in contact with said body-surface contact portion;

an indirect temperature detector disposed at a position that is adjacent to said heat conducting member and that is spaced apart from said body-surface contact portion, said indirect temperature detector measuring temperature at the position spaced apart from said body-surface contact portion;
a first storage portion storing information about hemoglobin concentration and hemoglobin oxygen saturation in blood;
a first storage portion for storing groups of measurement value sets or a calculation equation prepared for each of said groups of measurement value sets, said groups of measurement value sets being obtained by classifying a plurality of sets of measurement values obtained in advance by said heat amount measuring portion and said oxygen supply volume measuring portion by a statistical process;
a second storage portion for storing said first calculation equation;
a calculation portion for calculating a blood sugar level by using the outputs from said indirect temperature detector, said ambient temperature detector, said radiant heat detector, and said first calculation equation stored in said second storage portion; and
a display portion for displaying the blood sugar level outputted from said calculating portion.

The invention further provides a blood sugar level measuring apparatus comprising:

a heat amount measurement portion for measuring a plurality of temperatures derived from a body surface and obtaining information used for calculating the amount of heat transferred by convection and the amount of heat transferred by radiation, both related to the dissipation of heat from said body surface;
an oxygen amount measuring portion for obtaining information about blood oxygen level;
a storage portion for storing a relationship between parameters corresponding to said plurality of temperatures and blood oxygen amount and blood sugar levels;
a calculating portion which converts a plurality of measurement values fed from said heat amount measuring portion and said oxygen amount measurement portion into said parameters, and computes a blood sugar level by applying said parameters to said relationship stored in said storage portion;
a display portion for displaying the blood sugar level calculated by said calculating portion;
a communication interface; and
a control portion for replacing said relationship stored in said storage portion with said relationship acquired from said communication interface, wherein:

said oxygen level measurement portion includes a blood flow volume measurement portion for obtaining information about blood flow volume, and an optical measurement portion for obtaining hemoglobin concentration and hemoglobin oxygen saturation in blood, wherein said blood flow volume measurement portion includes:

a body-surface contact portion;
an indirect temperature detector for detecting the concentration at a position spaced apart from said body-surface contact portion; and
a heat conducting member connecting said body-surface contact portion and said indirect temperature detector.

The invention further provides a blood sugar level measuring apparatus comprising:

an ambient temperature measuring device for measuring ambient temperature;
a body-surface contact portion to which a body surface is brought into contact;
a radiant heat detector for measuring radiant heat from said body surface;
a heat conducting member disposed in contact with said body-surface contact portion;
an indirect temperature detector disposed at a position that is adjacent to said heat conducting member and that is spaced apart from said body-surface contact portion, said indirect temperature detector measuring temperature at the position spaced apart from said body-surface contact portion;
a light source for irradiating said body-surface contact portion with light of at least two different wavelengths;
a light detector for detecting reflected light produced as said light is reflected by said body surface;
a converter for converting outputs from said indirect temperature detector, said ambient temperature detector, said radiant heat detector and said light detector, into parameters;
a calculating portion in which a relationship between said parameters and blood sugar levels is stored in advance, and which calculates a blood sugar level by applying said parameters to said relationship;
a display portion for displaying the blood sugar level outputted from said calculating portion;

a communication interface; and
a control portion for replacing said relationship stored in said storage portion with said relationship acquired via said communication interface.

The invention further provides a blood sugar level measuring apparatus comprising:

an ambient temperature measuring device for measuring ambient temperature;
a body-surface contact portion to which a body surface is brought into contact;
a radiant heat detector for measuring radiant heat from said body surface;
a heat conducting member disposed in contact with said body-surface contact portion;
an indirect temperature detector disposed at a position that is adjacent to said heat conducting member and that is spaced apart from said body-surface contact portion, said indirect temperature detector measuring temperature at the position spaced apart from said body-surface contact portion;
a storage portion storing information about hemoglobin concentration and hemoglobin oxygen saturation in blood;
a converter for converting outputs from said indirect temperature detector, said ambient temperature detector, said radiant heat detector into a plurality of parameters;
a calculating portion in which a relationship between said parameters and blood sugar levels is stored in advance, and which calculates a blood sugar level by applying said parameters to said relationship;
a display portion for displaying the blood sugar level outputted from said calculating portion;
a communication interface; and
a control portion for replacing said relationship stored in said storage portion with said relationship acquired via said communication interface.

The invention further provides a system comprising:

a receiver portion adapted to receive a measurement data set including the temperature of a body surface, the temperature of a heat conducting member that is in contact with said body surface, the radiation temperature on said body surface, the values of a plurality of parameters calculated from ambient temperature, and a blood sugar level measured by a usual method;
a measurement data storage portion storing a plurality of measurement data sets received by said receiver portion;
a function data storage portion storing an average value and standard deviation value of each parameter, and a relation expression indicating the relationship between said parameters and blood sugar levels;
a processing portion for statistically processing the multiple data sets stored in said measurement data storage portion, determining a relation expression indicating the relationship between said parameters and blood sugar levels, and storing the relation expression in said function data storage portion; and
a transmitter portion for transmitting the average value and standard deviation value of each parameter stored in said function data storage portion and the relation expression indicating the relationship between said parameters and blood sugar levels.

In accordance with the invention, blood sugar levels can be determined in an non-invasive measurement with the same level of accuracy with that of the conventional invasive methods.

BRIEF DESCRIPTION OF THE DRAWINGS

Fig. 1 shows a model of the transfer of heat from a body surface to a block.
Fig. 2 shows changes in measurement values of temperatures $T_1$ and $T_2$ with time.
Fig. 3 shows an example of the measurement of a change in temperature $T_3$ with time.
Fig. 4 shows the relationship between measurement values obtained by various sensors and parameters derived therefrom.
Fig. 5 shows a top plan view and a lateral cross section of a non-invasive blood sugar level measuring apparatus according to the present invention.
Fig. 6 shows the flow of operation involving the finger.
Fig. 7 shows the flow of operation of the apparatus in response to button inputs.
Fig. 8 shows the details of a measurement portion.
Fig. 9 shows a system configuration for performing cluster analysis.
Fig. 10 shows a flowchart of cluster analysis.

Fig. 11 shows a flowchart of cluster analysis.

Fig. 12 schematically shows a server system according to the present invention.

Fig. 13 shows the plots of the calculated value of the glucose concentration using clustering and the measurement value of the glucose concentration obtained by the enzymatic electrode method.

Fig. 14 shows the plots of the calculated value of the glucose concentration without using clustering and the measurement value of the glucose concentration obtained by the enzymatic electrode method.

Fig. 15 shows the details of another example of the measurement portion.

Fig. 16 is a conceptual chart illustrating the location where data is stored in the apparatus.

Fig. 17 shows the plots of the glucose concentration value calculated by the invention and the glucose concentration value measured by the enzyme electrode method.

DESCRIPTION OF THE PREFERRED EMBODIMENTS

The invention will now be described by way of preferred embodiments thereof with reference made to the drawings.

Initially, the above-mentioned model will be described in more specific terms. Regarding the amount of heat dissipation, convective heat transfer, which is one of the main causes of heat dissipation, is related to temperature difference between the ambient (room) temperature and the body-surface temperature. The amount of heat dissipation due to radiation, another main cause of dissipation, can be determined by the Stefan-Boltzmann law, for example, which states that the amount of heat dissipation by radiation is proportional to the fourth power of the body-surface temperature. In another example, the radiant heat itself can be measured by a sensor. Thus, it can be seen that the amount of heat dissipation from the human body is related to the room temperature and the body-surface temperature. Another major factor related to the amount of heat production, the oxygen supply volume, is expressed as the product of hemoglobin concentration, hemoglobin oxygen saturation, and blood flow volume.

Initially, hemoglobin oxygen saturation and hemoglobin concentration will be discussed. In one example, the hemoglobin concentration can be measured based on the absorbance of light at the wavelength (iso-absorption wavelength) at which the molar absorption coefficient of the oxy-hemoglobin and that of the reduced (deoxy-) hemoglobin are equal. The hemoglobin oxygen saturation can be measured by measuring the absorbance of the iso-absorption wavelength and at least one other wavelength at which the ratio of the molar absorption coefficient of the oxy-hemoglobin to that of the reduced (deoxy-) hemoglobin is known, and then solving simultaneous equations. In another example, the hemoglobin oxygen saturation can be measured as follows. The absorbance at which the molar absorption coefficient of oxy-hemoglobin and that of deoxy-hemoglobin are equal, and the absorbance at least one other wavelength at which the ratio of the molar absorption coefficient of oxy-hemoglobin to that of deoxy-hemoglobin is known are measured. At the same time, hemoglobin oxygen saturation is measured using existing equipment. The relationship between the absorbance and hemoglobin oxygen saturation is subjected to multivariate analysis, and a calibration line is obtained that indicates the relationship between hemoglobin oxygen saturation and the absorbance at the aforementioned wavelengths. Thus, the hemoglobin concentration and the hemoglobin oxygen saturation can be obtained by measuring absorbance at least two wavelengths.

The rest is the blood flow volume, which can be measured by various methods. One example will be described below.

Fig. 1 shows a model for the description of the transfer of heat from the body surface to a solid block with a certain heat capacity as the block is brought into contact with the body surface for a certain time and then separated. The block is made of resin such as plastic or vinyl chloride. In the illustrated example, attention will be focused on the chronological variation of a temperature $T_1$ of a portion of the block in contact with the body surface, and the chronological variation of a temperature $T_2$ at a point on the block away from the body surface. The blood flow volume can be estimated by monitoring mainly the chronological variation of the temperature $T_2$ (at the spatially distant point on the block). The details will be described later.

Before the block comes into contact with the body surface, the temperatures $T_1$ and $T_2$ at the two points of the block are equal to the room temperature $T_r$. When a body-surface temperature $T_s$ is higher than the room temperature $T_r$, the temperature $T_1$ swiftly rises as the block comes into contact with the body surface, due to the transfer of heat from the skin, and it approaches the body-surface temperature $T_s$. On the other hand, the temperature $T_2$, which is lower than the temperature $T_1$ due to the dissipation of the heat conducted through the block from its surface, is damped and rises more gradually than the temperature $T_1$. The chronological variation of the temperatures $T_1$ and $T_2$ depends on the amount of heat transferred from the body surface to the block, which in turn depends on the blood flow volume in the capillary blood vessels under the skin. If the capillary blood vessels are regarded as a heat exchanger, the coefficient of heat transfer from the capillary blood vessels to the surrounding cell tissues is given as a function of the blood flow volume. Thus, by measuring the amount of heat transfer from the body surface to the block by monitoring the chronological variation of the temperatures $T_1$ and $T_2$, the amount of heat transmitted from the capillary blood vessels to the cell tissues can be estimated, which in turn makes it possible to estimate the blood flow volume.

Fig. 2 shows the chronological variation of the measured values of the temperature $T_1$ at the portion of the block in contact with the body surface and the temperature $T_2$ at the point on the block away from the body-surface contact position. As the block comes into contact with the body surface, $T_1$ swiftly rises, and it gradually drops as the block is brought out of contact.

Fig. 3 shows the chronological variation of the measured value of a temperature $T_3$ measured by a radiation temperature detector. As the temperature $T_3$ measured is that due to the radiation from the body surface, this sensor can more sensitively react to temperature changes than other sensors. Because radiation heat propagates as an electromagnetic wave, it can transmit temperature changes instantaneously. Thus, as shown in Fig. 7, by providing the radiation temperature detector near the position where the block is in contact with the body surface in order to detect the radiant heat from the body surface, contact start time $t_{start}$ and contact end time $t_{end}$ of contact between the block and body surface can be detected based on a change in temperature $T_3$. For example, when a temperature threshold value is set as shown in Fig. 3, it can be determined that contact start time $t_{start}$ is when the temperature threshold value is exceeded, and contact end time $t_{end}$ is when the measured temperature drops below the temperature threshold value. The temperature threshold value may be set at 32°C, for example.

Then, the $T_1$ measured value between $t_{start}$ and $t_{end}$ is approximated by an S curve, such as a logistic curve. A logistic curve is expressed by the following equation:

$$T = \frac{b}{1+c \times \exp(-a \times t)} + d$$

where T is temperature, and t is time.

The measured value can be approximated by determining factors a, b, c, and d by the non-linear least-squares method. For the resultant approximate expression, T is integrated between time $t_{start}$ and time $t_{end}$ to obtain a value $S_1$.

Similarly, an integrated value $S_2$ is calculated from the $T_2$ measured value. The smaller the $(S_1 - S_2)$ is, the larger the amount of transfer of heat from the finger surface to the position of $T_2$. $(S_1 - S_2)$ becomes larger with increasing finger contact time $t_{cont}$ $(=t_{end} - t_{start})$. Thus, $a_5/(t_{cont} \times (S_1 - S_2))$ is designated as a parameter $X_5$ indicating the volume of blood flow, where $a_3$ is a proportionality coefficient.

It will be seen from the above description that the measured quantities necessary for the determination of blood glucose concentration by the aforementioned model are the room temperature (ambient temperature), body surface temperature, temperature changes in the block in contact with the body surface, the temperature due to radiation from the body surface, and the absorbance of at least two wavelengths.

Fig. 4 shows the relationships between the measured values provided by various sensors and the parameters derived therefrom. A block is brought into contact with the body surface, and chronological changes in the two kinds of temperatures $T_1$ and $T_2$ are measured by two temperature sensors provided at two locations of the block. Separately, the radiation temperature $T_3$ on the body surface and the room temperature $T_4$ are measured. Absorbance $A_1$ and $A_2$ are measured at at least two wavelengths related to the absorption of hemoglobin. The temperatures $T_1$, $T_2$, $T_3$, and $T_4$ provide parameters related to the volume of blood flow. The temperature $T_3$ provides a parameter related to the amount of heat transferred by radiation. The temperatures $T_3$ and $T_4$ provide parameters related to the amount of heat transferred by convection. Absorbance $A_1$ provides a parameter relating to hemoglobin concentration. Absorbance $A_1$ and $A_2$ provide parameters relating to hemoglobin oxygen saturation.

Hereafter, an example of the apparatus for non-invasively measuring blood sugar levels according to the principle of the invention will be described.

Fig. 5 shows a top plan view of the non-invasive blood sugar level measuring apparatus according to the invention. While in this example the skin on the ball of the fingertip is used as the body surface, other parts of the body surface may be used.

On the upper surface of the apparatus are provided an operating portion 11, a measurement portion 12 where the finger to be measured is to be placed, and a display portion 13 for displaying the result of measurement, the state of the apparatus, measured values, and so on. The operating portion 11 includes four push buttons 11 a to 11d for operating the apparatus. The measurement portion 12 has a cover 14 which, when opened (as shown), reveals a finger rest portion 15 with an oval periphery. The finger rest portion 15 accommodates an opening end 16 of a radiation temperature sensor portion, a contact temperature sensor portion 17, and an optical sensor portion 18. The apparatus is also equipped with a rewritable storage portion 100 and an interface portion 110, the use of which will be described later.

Fig. 6 shows the operation procedure for the apparatus. As a button in the operation portion is pressed and the apparatus is turned on, the LCD displays "WARMING UP," during which the electronic circuitry in the apparatus is warmed up. Simultaneously, a check program is activated to automatically check the electronic circuitry. After the end of "WARMING UP," the LCD portion displays "PLACE FINGER." As the finger is placed on the finger rest portion, the LCD portion displays a countdown. When the countdown is over, the LCD portion displays "RELEASE FINGER." As the finger is released from the finger rest, the LCD displays "DATA PROCESSING," followed by the display of a blood sugar level. The thus displayed blood sugar level is stored in an IC card, together with the date and time. The subject reads the displayed blood sugar level and then presses a button in the operation portion. Approximately one minute later, the LCD portion displays "PLACE FINGER," indicating that the apparatus is now ready for the next measurement.

If the button 11d is pressed to enter into the measurement mode, the LCD portion displays "ENTER TIME DIVISION," as shown in Fig. 11. The subject then presses button 11b or 11c to select one of "BEFORE BREAKFAST," "AFTER BREAKFAST," "BEFORE LUNCH," "AFTER LUNCH," "BEFORE SUPPER," "AFTER SUPPER," AND "BEDTIME," and finalizes the selected time division by pressing the button 11d. After approximately 30 seconds layer, the LCD portion displays "PLACE FINGER." As the subject places his or her finger on the finger rest portion, the LCD portion displays a countdown. After the end of countdown, the LCD portion displays "RELEASE FINGER." As the subject releases his or her finger from the finger rest portion, the LCD portion displays "DATA PROCESSING" and then the blood sugar level that has been calculated.

If (1) the calculated blood sugar level is below 70 mg/dL, the LCD portion displays a warning message in order to warn the subject that he or she is in a state of hypoglycemia, with a beep of a buzzer. The LCD also displays a warning statement and activate the buzzer in the following cases: (2) the time division is before breakfast and the calculated blood sugar level is above 126 mg/dL; (3) the time division is after meal and the calculated blood sugar level is above 200 nm/dL; (4) the time division is before meal and the calculated blood sugar level is above a before-meal warning value that has been set by the subject; (5) the time division is after meal and the calculated blood sugar level is above a bedtime warning value that has been set by the subject. Thereafter, the displayed blood sugar level is stored in an IC card, together with the date of measurement and the time division. The subject then reads the displayed blood sugar level and presses the button 11d in the operation portion to return to the menu screen.

Fig. 12 shows the flow of operation of a warning-value setting portion in terms of software. As the subject presses the button 11a on the menu screen shown in Fig. 10, the software reads the before-meal warning value, after-meal warning value, and bedtime warning value from the IC card that have been previously set, and then stores them in the RAM. The software then causes a message "ENTER BEFORE-MEAL WARNING VALUE" to appear on the LCD, on which the previously set before-meal warning value is displayed. The value can be increased by pressing button 11c, and decreased by pressing button 11b. The subject thus adjusts the before-meal warning value by means of the buttons 11b and 11c, and then finalizes the before-meal warning value by pressing button 11d and proceeds to the screen for setting the after-meal warning value. In a similar manner, the after-meal warning value and the bedtime warning value are set, and then the software writes the finalized before-meal warning value, after-meal warning value and bedtime warning value into the IC card. After the writing into the IC card is completed, the software returns to the menu screen.

Fig. 13 and 14 show the flow of operation of a warning control portion in terms of software. After the blood sugar level is calculated, the software reads the before-meal blood sugar level, before-meal warning value, and before-breakfast warning value from the IC card and then stores them in RAM. If the calculated blood sugar level is below 70 mg/dL, the software displays "Blood sugar level is below 70 mg/dL" on the LCD along with the calculated blood sugar level that is already displayed, and repeats an ON/OFF control of the buzzer three times. If the time division is before meal, namely if it is either before breakfast, before lunch, or before supper, and if the calculated blood sugar level is more than 126 mg/dL, the software displays a warning message "Fasting blood sugar level is over 126 mg/dL" on the LCD along with the calculated blood sugar level that is displayed, and repeats the ON/OFF control of the buzzer three times. Fig. 15 shows how this is displayed on the LCD. If the time division is after meal, namely if it is either after breakfast, after lunch, or after supper, and if the calculated blood sugar level is over 200 mg/dL, the software displays a warning message "Nonfasting blood sugar level is over 200 mg/dL" on the LCD along with the calculated blood sugar level that is already displayed, and repeats the ON/OFF control of the buzzer three times.

If none of the above conditions is met, the software compares the calculated blood sugar level with the warning value set by the subject. First, if the time division is before meal, namely either before breakfast, before lunch, or before supper, and if the calculated blood sugar level exceeds the before-meal warning value, the software displays a warning message "Fasting blood sugar level is over XXX mg/dL" on the LCD along with the calculated blood sugar level that is already displayed, and repeats the ON/OFF control of the buzzer three times. In the space XXX, there is entered the before-meal warning. If the time division is after meal, namely either after breakfast, after lunch, or after supper, and if the calculated blood sugar level exceeds the after-meal warning value, the software displays a warning message "Nonfasting blood sugar level is over YYY mg/dL" on the LCD, along with the calculated blood sugar level that is already displayed, and repeats the ON/OFF control of the buzzer three times. In the space YYY, there is entered the after-meal warning value. If the time division is bedtime and if the calculated blood sugar level is more than the bedtime warning value, the software displays a warning message "Bedtime blood sugar level is over ZZZ mg/dL" on the LCD along with the calculated blood sugar level that is already displayed, and repeats the ON/OFF control three times. In the space XXX, there is entered the bedtime warning value. After leaving the warning control portion, the software writes measurement data consisting of the date of measurement, time division, and blood sugar level data.

Fig. 16 shows the flow of operation of a history display portion. In the menu screen of Fig. 10, as the subject presses button 11c, the software reads the measurement data including the date of measurement, time division and blood sugar level data for the past 210 sessions, and stores the data in RAM. The software then displays a message "Enter time division" on the LCD portion, as shown in Fig. 11. The subject presses button 11b or 11c to select one of "BEFORE BREAKFAST," "AFTER BREAKFAST," "BEFORE LUNCH," "AFTER LUHCN," "BEFORE SUPPER," "AFTER SUPPER," AND "BEDTIME" and finalizes the selected time division by pressing button 11d. Thereafter, the software displays the latest measurement data of the selected time division on the LCD, and then waits for the input via button 11a, 11b or 11c from the subject. If the button 11c is pressed, the software displays previous measurement data in the same time division. If the button 11b is pressed, the software displays subsequent measurement data in the same time division. If the button 11a is pressed, the software returns to the menu screen. Fig. 17 shows an example of the history data displayed on the LCD. The history data on a time-division basis thus provides useful targets when the subject intends to control the amount of insulin dosage and the amount of meal for breakfast, lunch, and supper.

Fig. 18 shows a functional block diagram of the apparatus according to the embodiment. The apparatus runs on battery 41. Signals measured by sensor portion 48 including temperature and optical sensors is fed to analog/digital converters AD1 to AD5 provided for individual signals and is converted into digital signals. A microprocessor 55 has built inside a ROM for storing software. An LED selecting LSI 19, under the control of microprocessor 55, is adapted to cause two light-emitting diodes, which are the light source of the optical sensor, to emit light in a time-divided manner. Peripheral circuits to microprocessor 55 include analog-digital converters AD1 to AD5, LCD 13, LED-selecting LSI 19, RAM 42, IC card 43, and realtime clock 45. Microprocessor 55 can access any of these via bus line 44. Push buttons 11 a to 11d are connected to microprocessor 55. A buzzer 56 is also connected to microprocessor 55. The buzzer 56 can be turned on or off by microprocessor 55.

Fig. 7 shows the details of the measurement portion. Fig. 7(a) is a top plan view, (b) is a cross section taken along line XX of (a), and (c) is a cross section taken along YY of (a).

First, temperature measurement by the non-invasive blood sugar level measuring apparatus according to the invention will be described. A thin plate 21 of a highly heat-conductive material, such as gold, is disposed on a portion where a measured portion (ball of the finger) is to come into contact. A bar-shaped heat-conductive member 22 made of a material with a heat conductivity lower than that of the plate 21, such as polyvinylchloride, is thermally connected to the plate 21 and extends into the apparatus. The temperature sensors include a thermistor 23, which is an adjacent temperature detector with respect to the measured portion for measuring the temperature of the plate 21. There is also a thermistor 24, which is an indirect temperature detector with respect to the measured portion for measuring the temperature of a portion of the heat-conducting member away from the plate 21 by a certain distance. An infrared lens 25 is disposed inside the apparatus at such a position that the measured portion (ball of the finger) placed on the finger rest portion 15 can be seen through the lens. Below the infrared lens 25, there is disposed a pyroelectric detector 27 via an infrared radiation-transmitting window 26. Another thermistor 28 is disposed near the pyroelectric detector 27.

Thus, the temperature sensor portion of the measurement portion has four temperature sensors, and they measure four kinds of temperatures as follows:

(1) Temperature on the finger surface (thermistor 23): $T_1$
(2) Temperature of the heat-conducting member (thermistor 24): $T_2$
(3) Temperature of radiation from the finger (pyroelectric detector 27): $T_3$
(4) Room temperature (thermistor 28): $T_4$

The optical sensor portion 18 will be described. The optical sensor portion measures the hemoglobin concentration and hemoglobin oxygen saturation for obtaining the oxygen supply volume. For measuring the hemoglobin concentration and hemoglobin oxygen saturation, absorbance must be measured at at least two wavelengths. Fig. 7 (c) shows an example of an arrangement for performing the two-wavelength measurement using two light sources 33 and 34 and one detector 35.

Inside the optical sensor portion 18, there are disposed the end portions of two optical fibers 31 and 32. The optical fiber 31 is for irradiating light, and the optical fiber 32 is for receiving light. As shown in Fig. 7(c), the optical fiber 31 is connected to branch fibers 31a and 31b at the ends of which light-emitting diodes 33 and 34 with two different wavelengths are provided. At the end of the optical fiber 32, there is provided a photodiode 35. The light-emitting diode 33 emits light of a wavelength 810 nm. The light-emitting diode 34 emits light of a wavelength 950 nm. The wavelength 810 nm is the iso-absorption wavelength at which the molar absorption coefficients of oxy-hemoglobin and reduced (deoxy-) hemoglobin are equal. The wavelength 950 nm is the wavelength at which the difference in molar absorption coefficients between the oxy-hemoglobin and the reduced hemoglobin is large.

The two light-emitting diodes 33 and 34 emit light in a time-divided manner. The light emitted by the light-emitting diodes 33 and 34 is irradiated via the light-emitting optical fiber 31 onto the finger of the subject. The light with which the finger is irradiated is reflected by the finger skin, incident on the light-receiving optical fiber 32, and then detected by the photodiode 35. When the light with which the finger is irradiated is reflected by the finger skin, some of the light penetrates through the skin and into the tissue, and is then absorbed by the hemoglobin in the blood flowing in capillary blood vessels. The measurement data obtained by the photodiode 35 is reflectance R, and the absorbance is approximated by log (1/R). Irradiation is conducted with light of the wavelengths 810 nm and 950 nm, and R is measured for each, and then log (1/R) is calculated, thereby measuring absorbance $A_1$ for wavelength 810 nm and absorbance $A_2$ for wavelength 950 nm.

When the reduced hemoglobin concentration is [Hb], and the oxy-hemoglobin concentration is [$HbO_2$], absorbance $A_1$ and $A_2$ are expressed by the following equations:

$$\begin{aligned} A_1 &= a \times ([Hb] \times A_{Hb}(810nm) + [HbO_2] \times A_{HbO_2}(810nm)) \\ &= a \times ([Hb] + [HbO_2]) \times A_{HbO_2}(810nm) \end{aligned}$$

$$\begin{aligned} A_2 &= a \times ([Hb] \times A_{Hb}(950nm) + [HbO_2] \times A_{HbO_2}(950nm)) \\ &= a \times ([Hb] + [HbO_2]) \times ((1 - \frac{[HbO_2]}{[Hb] + [HbO_2]}) \times A_{Hb}(950nm) + \frac{[HbO_2]}{[Hb] + [HbO_2]} \times A_{HbO_2}(950nm)) \end{aligned}$$

$A_{Hb}$ (810 nm) and $A_{Hb}$ (950 nm), and $A_{HbO2}$ (810 nm) and $A_{HbO2}$ (950 nm) are molar absorption coefficients of reduced hemoglobin and oxy-hemoglobin, respectively, and are known at the respective wavelengths. Sign a is a proportional coefficient. Based on the above equations, the hemoglobin concentration ([Hb] + [$HbO_2$]) and the hemoglobin oxygen saturation {[$HbO_2$] / ([Hb] + [$HbO_2$])} can be determined as follows:

$$[Hb] + [HbO_2] = \frac{A_1}{a \times A_{HbO_2}(810nm)}$$

$$\frac{[HbO_2]}{[Hb] + [HbO_2]} = \frac{A_2 \times A_{HbO_2}(810nm) - A_1 \times A_{Hb}(950nm))}{A_1 \times (A_{HbO_2}(950nm) - A_{Hb}(950nm))}$$

While in the above example the hemoglobin concentration and hemoglobin oxygen saturation are measured by measuring absorbance at two wavelengths, it is possible to reduce the influence of interfering components and increase measurement accuracy by measuring at three or more wavelengths.

Fig. 8 is a conceptual chart illustrating the flow of data processing in the apparatus. The apparatus according to the present example is equipped with five sensors, namely thermistor 23, thermistor 24, pyroelectric detector 27, thermistor 28 and photodiode 35. The photodiode 35 measures the absorbance at wavelength 810 nm and the absorbance at wavelength 950 nm. Thus, six kinds of measurement values are fed to the apparatus.

Five kinds of analog signals are supplied via amplifiers A1 to A5 and digitally converted by analog/digital converters AD1 to AD5. Based on the digitally converted values, parameters $x_i$ (i=1-10) are calculated. The following are specific descriptions of $x_i$ (where $a_1$ to $a_7$ are proportionality coefficients):

Parameter suggesting heat radiation

$x_1 = a_1 \times (T_3)^4$ : From the Stefan-Boltzmann law.

$x_2 = T_3$ : Radiant heat is directly detected by a sensor.

Parameter suggesting heat convection

$x_3 = a_2 \times (T_4 - T_3)$ Convection is determined from room temperature and radiant heat from finger.

$x_4 = a_3 \times (T_4 - T_1)$ Convection is determined from room temperature and the temperature on the finger surface.

Parameter suggesting hemoglobin concentration

$$x_5 = a_4 \left( \frac{A_1}{a \times A_{HbO_2}(810nm)} \right)$$

Parameter suggesting hemoglobin oxygen saturation

$$x_6 = a_5 \times \left( \frac{A_2 \times A_{HbO_2}(810nm) - A_1 \times A_{Hb}(950nm))}{A_1 \times (A_{HbO_2}(950nm) - A_{Hb}(950nm))} \right):$$

$x_7 = A_1$: Determined from a polynomial expression for determining hemoglobin oxygen saturation.
$x_8 = A_2$ : Determined from a polynomial expression for determining hemoglobin oxygen saturation.

Parameter suggesting blood flow volume

$$x_9 = a_6 \times \left( \frac{1}{t_{CONT} \times (S_1 - S_2)} \right):$$

Determined from the amount of temperature shift.

$x_{10} = a_7 \times \frac{dT_2}{dt}$ : Rate of change in finger surface temperature.

As described above, there are a plurality of parameters suggesting certain physical quantities. In the present scheme, physiological metabolism of each individual is measured to determine glucose concentration. The metabolism activities of each individual are influenced by such factors as sec, age, weight and medical history, and therefore which parameter affects the glucose concentration varies from one individual to another. Possibly, it is better to combine two or more parameters. These decisions can be made by conducting a multivariate analysis, determining the correlation between glucose concentrations, and then determining the ratio of contribution of each parameter. For this analysis, various analysis methods can be utilized, such as linear multiple regression analysis, principal component regression analysis, and PLS regression analysis. In the following example, the linear regression analysis is used.

The parameters suggesting blood flow volume or convection are not limited to those mentioned above, and more parameters may be added. For example, in the event that a novel parameter suggesting blood flow volume is devised, the validity of the novel parameter can be verified by conducting a statistical analysis and evaluating the ratio of contribution of the parameter.

Then, normalized parameters are calculated from mean values and standard deviations of parameters $x_i$ obtained for each patient from actual data from large numbers of able-bodied people and diabetic patients. A normalized parameter $X_i$ (where i=1 to 10) is calculated from each parameter $x_i$ according to the following equation:

$$X_i = \frac{x_i - \bar{x}_i}{SD(x_i)}$$

where

$x_i$: parameter
$\bar{x}_i$ : mean value of the parameter
$SD(x_i)$: standard deviation of the parameter

Calculations are conducted to convert the above 10 normalized parameters $X_i$ (i=1 to 10) into a glucose concentration that is to be eventually displayed. Programs necessary for computations are stored in ROM and/or rewritable storage portion built inside the microprocessor in the apparatus. Memory areas necessary for computations are ensured in a RAM built inside the apparatus. The results of the calculations are displayed on the LCD portion.

The accuracy of the average value of parameter $x_i$ depends on the number of the able-bodied persons and diabetic patients from which the actual data has been obtained. This is due to the fact that how accurately the average value obtained from a number n of samples represents the average value of the population depends on the number n of samples for statistical reasons. For example, an average value obtained from 100 samples indicates the average value of the population more accurately and with better expected accuracy of the average value, than an average value obtained from dozens of samples. Thus, it is necessary to collect data from a sufficient number of subjects if the accuracy of the average value is to be increased.

The ROM stores, as a constituent element of the program necessary for the computations, a calculation process for determining glucose concentration C in particular. In the rewritable storage portion, coefficients $a_i$ (i=0 to

10) and the parameters used are stored. These coefficients and parameters are determined as follows. First, data is acquired from a large number of able-bodied persons and diabetic patients. It is necessary here to gather a sufficient amount of data in order to ensure that an accurate multiple regression equation indicating the relationship between the normalized parameters and glucose concentration C can be prepared for each patient. Thereafter, the multiple regression equation indicating the relationship between the normalized parameters and glucose concentration C is prepared according to the following procedure for each patient.

First, glucose concentration C is expressed by the following equation (1). The procedure for determining $a_i$ (i = 0 to 10) is as follows:

(1) A multiple regression equation is created that indicates the relationship between the normalized parameters and the glucose concentration C.
(2) Normalized equations (simultaneous equations) relating to the normalized parameters are obtained from an equation obtained by the least-squares method.
(3) Values of coefficient $a_i$ (i=0 to 10) are determined from the normalized equations and then substituted into the multiple regression equation.

Initially, the regression equation (1) indicating the relationship between the glucose concentration C and the normalized parameters $X_1$ to $X_{10}$ is formulated.

$$\begin{aligned} C &= f(X_1,X_2,X_3,X_4,X_5,X_6,X_7,X_8,X_9,X_{10}) \\ &= a_0 + a_1X_1 + a_2X_2 + a_3X_3 + a_4X_4 + a_5X_5 + a_6X_6 + a_7X_7 + a_8X_8 + a_9X_9 + a_{10}X_{10} \quad ......(1) \end{aligned}$$

Then, the least-squares method is employed to obtain a multiple regression equation that would minimize the error with respect to a measured value $C_i$ of glucose concentration according to an enzyme electrode method. When the sum of squares of the residual is D, D is expressed by the following equation (2):

$$\begin{aligned} D &= \sum_{i=1}^{n} d_i^{\,2} \\ &= \sum_{i=1}^{n} (C_i - f(X_{i1},X_{i2},X_{i3},X_{i4},X_{i5},X_{i6},X_{i7},X_{i8},X_{i9},X_{i10}))^2 \\ &= \sum_{i=1}^{n} \{C_i - (a_0 + a_1X_{i1} + a_2X_{i2} + \cdots + a_9X_{i9} + a_{10}X_{i10})\}^2 \quad ......(2) \end{aligned}$$

The sum of squares of the residual D becomes minimum when partial differentiation of equation (2) with respect to $a_0$, $a_1$, ..., $a_{10}$ gives zero. Thus, we have the following equations:

$$\frac{\partial D}{\partial a_0} = -2\sum_{i=1}^{n}\{C_i - (a_0 + a_1X_{i1} + \cdots + a_9X_{i9} + a_{10}X_{i10})\} = 0$$

$$\frac{\partial D}{\partial a_1} = -2\sum_{i=1}^{n}X_{i1}\{C_i - (a_0 + a_1X_{i1} + \cdots a_9X_{i9} + a_{10}X_{i10})\} = 0$$

$$\frac{\partial D}{\partial a_2} = -2\sum_{i=1}^{n}X_{i2}\{C_i - (a_0 + a_1X_{i1} + \cdots + a_9X_{i9} + a_{10}X_{i10})\} = 0$$

$$\cdots\cdots\cdots\cdots$$

$$\frac{\partial D}{\partial a_9} = -2\sum_{i=1}^{n}X_{i9}\{C_i - (a_0 + a_1X_{i1} + \cdots + a_9X_{i9} + a_{10}X_{i10})\} = 0$$

$$\frac{\partial D}{\partial a_{10}} = -2\sum_{i=1}^{n}X_{i10}\{C_i - (a_0 + a_1X_{i1} + \cdots + a_9X_{i9} + a_{10}X_{i10})\} = 0 \quad ......(3)$$

When the mean values of C and $X_1$ to $X_{10}$ are $C_{mean}$ and $X_{1mean}$ to $X_{10mean}$, respectively, since $X_{imean}$=0 (i=1 to 10), equation (1) yields equation (4) thus:

$$a_0 = C_{mean} - a_1X_{1mean} - a_2X_{2mean} - \cdots - a_9X_{9mean} - a_{10}X_{10mean}$$
$$= C_{mean} \quad ......(4)$$

The variation and covariation between the normalized parameters are expressed by equation (5). Covariation between the normalized parameter $X_i$ (i=1 to 10) and C is expressed by equation (6).

$$S_{ij} = \sum_{k=1}^{n}(X_{ki} - X_{imean})(X_{kj} - X_{jmean}) = \sum_{k=1}^{n}X_{ki}X_{kj} \quad (i, j = 1,2,..10) \quad ......(5)$$

$$S_{iC} = \sum_{k=1}^{n}(X_{ki} - X_{imean})(C_k - C_{mean}) = \sum_{k=1}^{n}X_{ki}(C_k - C_{mean}) \quad (i = 1,2,..10) \quad ......(6)$$

Substituting equations (4), (5), and (6) into equation (3) and rearranging yields simultaneous equations (normalized equations) (7). Solving equations (7) yields $a_1$ to $a_{10}$.

$$a_1S_{11} + a_2S_{12} + \cdots + a_9S_{19} + a_{10}S_{110} = S_{1C}$$
$$a_1S_{21} + a_2S_{22} + \cdots + a_9S_{29} + a_{10}S_{210} = S_{2C}$$
$$\cdots\cdots$$
$$a_1S_{91} + a_2S_{92} + \cdots + a_9S_{99} + a_9S_{99} = S_{4C}$$
$$a_1S_{101} + a_2S_{102} + \cdots + a_9S_{109} + a_{10}S_{1010} = S_{5C} \quad ......(7)$$

Constant term $a_0$ is obtained by means of equation (4). Thus, the multiple regression line for each patient in a base group of patients can be determined.

Next, in order to obtain a generalized calibration line from the multiple regression lines obtained for the individual patients, a cluster analysis is performed on the obtained multiple regression lines. By this, adjacent calibration lines can be clustered and groups of patients can be created. In cluster analysis, the degree of similarity among multiple regression lines must be evaluated. Examples of the evaluation methods include Euclidean distance, Minkowski distance, and Mahalanobis distance. In the present embodiment, the Mahalanobis distance is used.

The Mahalanobis distance shows the distance between a given point and the center of gravity of a set. If a parameter of measurement data about a patient takes on a specific value ($x_{10}$, ..., $x_{p0}$), when the average of a certain cluster is ($\bar{x}_1,\cdots,\bar{x}_p$), and the covariance is $s_{jl}$, the Mahalanobis is expressed by

$$D^2 = \sum_{j=1}^{p} \sum_{l=1}^{p} \left(x_{j0} - \bar{x}_j\right)\left(x_{l0} - \bar{x}_l\right) s_{jl}$$

The Mahalanobis distance is used for discriminant analysis in statistics. The smaller the distance, the higher the probability that an obtained sample belongs to a known set.

Thereafter, a multiple regression line is obtained for each cluster by using all of the data of patients in a group, in order to obtain a generalized calibration line. The number of clusters depends on the number of items of obtained data and the desired accuracy of glucose concentration calculation. If there are sufficient amounts of obtained data, the number of clusters can be determined by the accuracy of glucose concentration calculation.

The multiple regression line for each cluster is a polynomial expression with 10 parameters. As mentioned above, the physiological metabolism varies from one individual to another, and the rate of contribution of a particular parameter is high in some individuals and low in others. While the 10-parameter polynomial expression may be used as is, by eliminating parameters with lower rates of contribution, the computation speed or the device for storing programs can be reduced. In the present embodiment, five parameters with lower rates of contribution are eliminated. When parameters with lower rates of contribution are eliminated, the multiple regression line for each cluster must be determined again by using the parameters used.

The method and an example of the procedure for assigning a cluster calibration line to a new patient will be described below. System configuration is shown in Fig. 9. Data flow is shown in Fig. 10.

(1) Multiple parameter data and a glucose concentration measurement value (enzymatic electrode method) are acquired from the new patient at the same time. The parameter data is stored in a storage portion 100. There are several procedures for measuring the parameter data and glucose concentration that are acquired here. For example, blood is sampled first and the glucose concentration is measured, and then parameter data is measured three times. Thereafter, a blood sample is taken and the glucose concentration is measured. By comparing the two glucose concentrations, glucose change during the acquisition of parameter data can be known, so that data indicating sharp glucose concentration changes can be avoided. If there is a sharp concentration change, the correspondence between parameter data and glucose concentration deteriorates, thereby affecting the cluster allocation. By measuring the parameter data a plurality of times, data due to an accidental measurement error can be identified and eliminated.

(2) The parameter data is sent via an interface 110 to calculation-equation selection means 140, which is a means of selecting a calculation equation for calculating a calibration line, namely glucose concentration. The glucose concentration corresponding to the parameter data is fed to calculation-equation selection means 140.

(3) The calculation-equation selection means determines a multiple regression line (calculation equation) for the new patient based on the obtained parameter data.

(4) The calculation equation selecting means determines to which cluster the obtained multiple regression line belongs to, according to discriminant analysis (analysis by calculations of the Mahalanobis distance, for example), using a database 150 of clustered data groups.

(5) A calculation equation corresponding to the thus determined cluster is received from the database of clustered calculation equations. Specifically, the received data is a coefficient $a_i$ (i=0 to 5) of the multiple regression line of the cluster, and the parameter used.

(6) Calculation-equation selection means 140 stores the data received from the database 150 of the clustered calculation equations and data groups in the rewritable storage portion 100 of the apparatus.

(7) In the actual measurement using the apparatus, glucose concentration C is calculated using a coefficient, parameters suitable for the patient which are stored in the storage portion 100 and normalized parameters X1 to X10 that were obtained from measurement values.

Alternatively, the procedure for the determination and analysis in steps (3) and (4) of the above procedure can be performed by different methods, as described below. Data flow for this case is shown in Fig. 11.

(1) Step (1) above is performed.
(2) Step (2) above is performed.
(3) Calculation equation selecting means 140 calculates the glucose concentration by substituting the obtained parameter data into all of the calculation equation in database 150 of the clustered calculation equations.
(4) The calculation equation selecting means determines to which cluster the calculated glucose concentration belongs to, using a correlation coefficient (the highest correlation coefficient is adopted) between the calculated glucose concentration and a reference glucose concentration.
(5) Steps (5) to (7) above are performed. Thus, the calculation equation selecting means can be implemented by several techniques and any of which may be used. Although in the above example the parameter data is sent to calculation equation selecting means 140 via interface 110, there may be provided the calculation equation selecting means inside the apparatus such that the coefficient $a_i$ (i=0 to 5) of the cluster multiple regression line and the parameters used can be obtained while communicating between the apparatus and the database.

The above-described procedures can be implemented at hospitals, pharmacies, or specialty shops. However, since glucose concentrations that have been measured by routine examination methods are required as reference values, there must be provided qualified staff capable of taking blood samples, such as doctors or nurses. For this reason, the procedures should be generally conducted in hospitals. If qualified staff are available, however, it could also be conducted at pharmacies or specialty shops.

The allocated clusters have certain expiration dates. This is due to the probable, gradual changes in physiological metabolism with the passage of time. Thus, the cluster determination must be performed periodically. This will lead to the actual diabetic patient's periodic visits to a doctor for guidance on how to control his or her blood sugar level, which will contribute to the maintenance or improvement of the symptom.

By conducting the cluster determination periodically, the parameters of each patient and the reference glucose concentrations can be collected from all of the patients on a periodic basis. Such data can be collected and a cluster analysis can be conducted again after a certain amount of data has been accumulated, resulting in more accurate clusters. For the collection of data in this case, a server system shown in Fig. 12 can be utilized. Further, by providing a data storage machine 160 between the blood sugar level measuring apparatus and a server 120 and installing the data storage machine at a hospital, pharmacy, or specialty shop, it becomes possible to collect field data. Data storage machine 160 acquires via interface portion 110 of the blood sugar level measuring apparatus the values of various parameters measured by the apparatus, and stores them as a data set, together with the blood sugar level of the subject measured by the enzymatic electrode method. The thus collected data is transmitted via communication means to server 120.

By simultaneously acquiring the medical history of the patients selected for the cluster analysis via questionnaires or from the doctor in charge, and investigating the disease states for each cluster, a certain cluster might indicate a specific disease state. Such an investigation could therefore provide data that can help the doctor with diagnosis.

In the above calculation methods, the accuracy of the average value of parameter $x_i$ and the values of $a_i$ (i=0 to 10) are dependent on the number of data items that are measured in advance. Accordingly, if the parameters measured by the blood sugar level measuring apparatus that is used by the patient can be stored in the apparatus, and if the parameters and the blood sugar level measured by the usual method (enzymatic electrode method) at the time of the measurement of the parameters can be collected, the measurement accuracy of the blood sugar level measuring apparatus can be improved. Hereafter, a method for that purpose is described.

In one example, the blood sugar level measuring apparatus is equipped with rewritable storage portion 100 in which the parameters that are calculated upon normal use of the apparatus can be stored. The storage portion may be formed by a memory device fixed inside the apparatus, such as a RAM or EEPROM. Alternatively, it may be an external memory device, such as a flash memory or EEPROM, that can be detached. The blood sugar level measuring apparatus is equipped with interface portion 110 for transmitting the thus stored parameters to server 120 where the measurement data used by the blood sugar level calculation equation in the current apparatus is stored (see Fig. 5). The interface portion may be a wireless interface such as wireless LAN, PHS, or infrared communications, or a cable interface such as USB, RS232C, or an analog terminal. The interface may be connected to a personal computer, a portable terminal, or a cell phone, for example.

Referring to Fig. 12, the data collection and analysis method will be described. This technique, in which measurement data is accumulated and the calibration line is renewed, is an effective method for improving the accuracy of the calibration line not only in cases where a plurality of calibration lines are prepared by cluster analysis, but also in cases where a single calibration line is applied for the entire subjects without conducting cluster analysis.

Server 120 comprises a transmission/reception portion 121 for receiving or transmitting data by communicating with the blood sugar level measuring apparatus or data storage machine 160 installed in a hospital or pharmacy or the like. It also includes a processing portion 123 for performing processes such as a calculation process, a measurement data storage portion 124 in which each parameter measured by the blood sugar level measuring apparatus and the blood sugar level measured by the enzymatic electrode method are stored as a single data set. Server 120 also includes a function data storage portion 125 for storing the average value and standard deviation values of each parameter, and coefficient $a_i$ (i=0 to 10) for regression equation (1). The data set and the function data may be stored in the same storage portion.

The various parameters and the glucose concentration measurement value (enzymatic electrode method) collected in data storage machine 160 or stored in storage portion 100 of the blood sugar level measuring apparatus are transmitted to server 120. The data received by a reception portion of server 120 is additionally stored in measurement data storage portion 124 via processing portion 123. When the number of the data items that have been added, or the number of the data items stored in measurement data storage portion 124, has reached a specified data number, processing portion 123 re-calculates the average value and standard deviation of each parameter and coefficient $a_i$ (i=0 to 10) of regression equation (1), using the entire data stored in measurement data storage portion 124. The resultant re-calculated values are stored in function data storage portion 125. Also, the average value and standard deviation of each parameter and coefficient $a_i$ (i=0 to 10) for regression equation (1), which are stored in function data storage portion 125, are updated. Then, the parameter average values and standard deviations and coefficient $a_i$ (i=0 to 10) that have been updated in function data storage portion 125 of server 120 are directly transmitted to the blood sugar level measuring apparatus or to data storage machine 160. As a result, the parameter average values and standard deviations and coefficient $a_i$ (i=0 to 10) stored in the blood sugar level measuring apparatus, or the parameter average values and standard deviations and coefficient $a_i$ (i=0 to 10) stored in the data storage machine are replaced with the updated parameter average value and coefficient $a_i$ (i=0 to 10). When the updated values are transmitted to the data storage machine, the updated data can be transferred to the blood sugar level measuring apparatus by connecting it to the data storage machine.

In the following, an example of the process of calculating parameter $X_1$ will be described. $X_1$ is parameter $x_1$ that has been normalized. If it is now assumed that the parameter distribution is normal, 95% of the normalized parameters take on a value between -2 and +2. When the value of parameter $x_1$ is $1.74 \times 10^3$, the average value of parameter $x_1$ is $1.75 \times 10^3$, and the standard deviation of parameter $x_1$ is 167, a specific normalized parameter can be obtained by the following equation:

$$X_1 = -0.06 = \frac{1.74\times10^3 - 1.75\times10^3}{167}$$

$\bar{x}_i$ : average value of parameter $=1.75 \times 10^3$

$SD(x_i)$ : stadard deviation of parameter =167

If the average value of the parameter in the above equation is the average value of 20 samples, the 95% confidence interval of the average value of $x_1$ can be determined as follows:

$1.75 \times 10^3 - 1.725 \times 167 <$ 95% confidence interval of $\bar{x}_1 <: 1.75 \times 10^3 + 1.725 \times 167$

If the average value is that of 40 samples, the 95% confidence interval of the average value of $x_1$ will be:

$1.75 \times 10^3 - 1.684 \times 167 <$ 95% confidence interval of $\bar{x}_1 <: 1.75 \times 10^3 + 1.684 \times 167$

Thus, the accuracy is improved, which is the same as the improvement of the accuracy of the normalized parameter. Further, if the number of samples is 60, the 95% confidence interval of the average value of $x_1$ will be:

$1.75 \times 10^3 - 1.671 \times 167 <$ 95% confidence interval of $\bar{x}_1 <: 1.75 \times 10^3 + 1.671 \times 167$

Thus, the accuracy is further improved.

In the following, an example of the process of calculating the glucose concentration will be described. Data was collected from 21 able-bodied persons and diabetic patients, using the present apparatus, and then multiple regression lines for estimating the glucose concentration were obtained based on calculated parameters. Then, cluster analysis was conducted to cluster adjacent lines into groups. The results are shown in Table 1.

Table 1

| Group | Subject Number |
|---|---|
| 1 | 2, 5, 8, 12, 14 |
| 2 | 4, 6, 9, 11 |
| 3 | 7, 10, 13, 18 |
| 4 | 1, 17, 19, 20 |
| 5 | 3, 15, 16, 21 |

From these results, four clusters were obtained. Table 2 shows the parameters used for individual clusters. For reference purpose, Table 2 also shows the five parameters adopted when a multiple regression analysis was conducted on the entire subjects as one group, without performing cluster analysis.

Table 2

| Cluster No. | | 1 | 2 | 3 | 4 | 5 |
|---|---|---|---|---|---|---|
| Heat radiation | X1 | O | O | O | O | X |
| | X2 | X | X | X | X | O |
| Heat convection | X3 | O | O | O | X | O |
| | X4 | X | X | X | O | X |
| Hemoglobin concentration | X5 | O | O | X | O | O |
| | X6 | X | X | O | X | X |
| Hemoglobin oxygen saturation | X7 | O | O | X | O | O |
| | X8 | X | X | O | X | X |
| Blood flow volume | X9 | O | X | O | O | O |
| | X10 | X | O | X | X | X |
| O: Adopted as a parameter for glucose concentration calculation<br>X: Not adopted as a parameter for glucose concentration calculation | | | | | | |

Next, data was collected from a new patient by measuring five times using the present apparatus. At the same time, the glucose concentration was measured by the enzymatic electrode method as a comparative method. The resultant data and glucose concentration were subjected to a multivariate analysis to obtain a multiple regression line. Then, the Mahalanobis distance between the resultant multiple regression line and each cluster was calculated, and the nearest cluster was taken as the cluster for the patient, which was cluster No. 3 in the present example. Thereafter, the coefficient of the determined cluster was stored in the rewritable storage portion. The following are the entire glucose calculation equations obtained by cluster analysis:

$$C_1 = 93.4+16.2\times X_1-19.4\times X_3 -23.4\times X_5 -20.5\times X_7 -27.2\times X_{10}$$

$$C_2 = 95.2+14.3\times X_1-18.2\times X_3-21.3\times X_6-18.75\times X_8-26.5\times X_9$$

$$C_3 = 97.1+15.1\times X_1-19.5\times X_4-24.2\times X_5 -21.2\times X_7-28.1\times X_9$$

$$C_4 = 98.2+20.7\times X_2-18.5\times X_3 -22.2\times X_5 -19.4\times X_7-30.5\times X_9$$

For reference, the following is the regression function obtained by performing a multiple regression analysis again on the five parameters for the entire subjects as one group:

$$C_T = 99.4 +18.3 \times X_1- 20.2 \times X_3-23.7 \times X_5 -22.0\times X_7 -25.9 \times X_9$$

Hereafter, the results of measurement by the conventional enzymatic electrode method, in which a blood sample is reacted with a reagent and the amount of resultant electrons is measured to determine blood sugar level, and those by an embodiment of the invention will be described.

When the glucose concentration was 89 mg/dL according to the enzymatic electrode method in an example of measured values for an able-bodied person, substituting normalized parameters $X_1$=-0.06, $X_2$=0.10, $X_3$=0.04, $X_4$=0.03, $X_5$=0.05, $X_6$=-0.07, $X_7$=-0.12, $X_8$=-0.16, $X_9$=0.10, and $X_{10}$=-0.08 obtained by measurement at the same time according to the inventive method into all of the above equation yield $C_1$=95 mg/dL, $C_2$=95 mg/dL, $C_3$=93 mg/dL, and $C_4$=98 mg/dL. The value without clustering is $C_T$=96 mg/dL.

Further, when the glucose concentration was 238 mg/dL according to the enzymatic electrode method in an example of measurement values for a diabetic patient, substituting normalized parameters $X_1$=1.15, $X_2$=0.93, $X_3$=-0.93, $X_4$=-1.31, $X_5$=-0.83, $X_6$=-0.84, $X_7$=-0.91, $X_8$=-0.50, $X_9$=-1.24, and $X_{10}$=-0.93 obtained by measurement at the same time according to all of the inventive method yields $C_1$=193 mg/dL, $C_2$=188 mg/dL, $C_3$=214 mg/dL, and $C_4$=210 mg/dL. The value without clustering is $C_T$=211 mg/dL.

Fig. 13 shows the plots of the results of measurement of three subjects, the vertical axis indicating the glucose concentration calculated by the present apparatus using a different regression function for each cluster, and the horizontal axis indicating the glucose concentration simultaneously measured by the enzymatic electrode method. The correlation coefficient is 0.934. When a line y=Ax+B (where y is the vertical axis and x is the horizontal axis) is fitted on each plot of the figure using the least squares method, A=0.992 and B=-6.07.

On the other hand, Fig. 14 shows the plots of the results of measurement of a total of 100 subjects, including 80 diabetic patients and 20 able-bodied persons, with the vertical axis indicating the glucose concentration measured by the present apparatus in which the regression function obtained by treating the entire subjects as one group is stored, and the horizontal axis indicating the glucose concentration simultaneously measured by the enzymatic electrode method. The correlation coefficient is 0.901. When a line y=Ex+F (where y is the vertical axis and x is the horizontal axis) is fitted on each plot of the figure by the least squares method, E=0.962 and F=8.15.

The closer the measurement value obtained by the present apparatus to that by the enzymatic electrode method, the closer the measurement accuracy of the present apparatus to that of the invasive method. Namely, in the plots of values in the above figures, the closer the correlation coefficient to 1, the higher the measurement accuracy of the present apparatus. Thus, the results shown in Figs. 13 and 14 reveal that higher measurement accuracy can be achieved by conducting the measurement using a different regression function for each group than by using the common regression function obtained from the measurement data about the subjects in the entire groups.

In the above-described embodiment, the parameters relating to blood hemoglobin concentration and blood hemoglobin oxygen saturation are obtained by spectroscopically measuring the hemoglobin in blood. However, the hemoglobin concentration is stable in persons without such symptoms as anemia, bleeding or erythrocytosis. The hemoglobin concentration is normally in the range between 13 to 18 g/dL for males and between 12 to 17 g/dL for females, and the range of variation of hemoglobin concentration from the normal values is 5 to 6%. Further, the weight of the term in the aforementioned formula for calculating blood sugar level is smaller than other terms. Therefore, the hemoglobin concentration can be treated as a constant without greatly lowering the measurement accuracy. Similarly, the hemoglobin oxygen saturation is stable between 97 to 98% if the person is undergoing aerial respiration at atmospheric pressure, at rest and in a relaxed state. Thus the hemoglobin concentration and the hemoglobin oxygen saturation can be treated as constants, and the oxygen supply volume can be determined from the product of the hemoglobin concentration constant, the hemoglobin oxygen saturation constant and the blood flow volume.

By treating the hemoglobin concentration and hemoglobin oxygen saturation as constants, the sensor arrangement for measuring blood sugar level can be simplified by removing the optical sensors, for example. Further, by eliminating the time necessary for optical measurement and the processing thereof, the procedure for blood sugar level measurement can be accomplished in less time.

Because the hemoglobin oxygen saturation takes on a stable value when at rest, in particular, by treating the hemoglobin concentration and hemoglobin oxygen saturation as constants, the measurement accuracy for blood sugar level measurement when at rest can be increased, and the procedure blood sugar level measurement can be accomplished in less time. By "when at rest" herein is meant the state in which the test subject has been either sitting on a chair or lying and thus moving little for approximately five minutes.

Hereafter, an embodiment will be described in which the blood hemoglobin concentration and blood hemoglobin oxygen saturation are treated as constants. This embodiment is similar to the above-described embodiment except that the blood hemoglobin concentration and blood hemoglobin oxygen saturation are treated as constants, and therefore the following description mainly concerns the differences from the earlier embodiment.

In the present embodiment, the hemoglobin concentration and hemoglobin oxygen saturation shown in Fig. 4 are not measured but treated as constants. Therefore, as shown in Fig. 15, the measurement portion of the present embodiment has the structure of the measurement portion of the earlier embodiment shown in Fig. 7 from which the light sources 33 and 34, photodiode 35 and optical fibers 31 and 32 are removed. Parameters used in the present embodiment are parameters $x_1$ and $x_2$ suggesting heat radiation, parameter $x_3$ and $x_4$ suggesting heat convection, and parameters $x_9$ and $x_{10}$ suggesting the oxygen supply volume. From these parameters, normalized parameters are calculated in the manner described above, and the glucose concentration is calculated based on three of the normalized parameters $X_i$, for example. During data processing, the step "CONVERSION OF OPTICAL MEASUREMENT DATA INTO NORMALIZED PARAMETERS" (see Fig. 8), which is necessary in the previous embodiment, can be omitted.

Fig. 16 shows a functional block diagram of the apparatus according to the embodiment. The apparatus runs on battery 41. A signal measured by sensor portion 48 including a temperature sensor is fed to analog/digital converters 44 (AD1 to AD4) provided for individual signals and is converted into a digital signal. Analog/digital converters AD1 to AD4, LCD 13 and RAM 42 are peripheral circuits for microprocessor 45. They are accessed by the microprocessor 45 via bus line 46. The push buttons 11a to 11d are connected to microprocessor 55. The microprocessor 45 includes the ROM for storing software. By pressing the buttons 11a to 11d, external instructions can be entered into microprocessor 45.

The ROM 47 included in the microprocessor 45 stores a program necessary for computations, i.e., it has the function of an arithmetic unit. The microprocessor 55 further includes a hemoglobin concentration constant storage portion 48 for storing hemoglobin concentration constants, and a hemoglobin oxygen saturation constant storage portion 49 for storing hemoglobin oxygen saturation constants. After the measurement of the finger is finished, the computing program calls optimum constants from the hemoglobin concentration storage portion 48 and hemoglobin oxygen saturation constant storage portion 49 and perform calculations. A memory area necessary for computations is ensured in the RAM 42 similarly incorporated into the apparatus. The result of computations is displayed on the LCD portion.

The ROM stores, as a constituent element of the program necessary for the computations, a function for determining glucose concentration C in particular. The function is defined as follows. C is expressed by a below-indicated equation (8), where $a_i$ (i=0, 1, 2, 3) is determined from a plurality of pieces of measurement data in advance according to the following procedure:

(1) A multiple regression equation is created that indicates the relationship between the normalized parameter and the glucose concentration C.
(2) Normalized equations (simultaneous equations) relating to the normalized parameter are obtained from an equation obtained by the least-squares method.
(3) Values of coefficient $a_i$ (i=0, 1, 2, 3) are determined from the normalized equation and then substituted into the multiple regression equation.

Initially, the regression equation (8) indicating the relationship between the glucose concentration C and the normalized parameters $X_1$, $X_2$, $X_3$ ($X_1$, $X_2$, $X_3$ are normalized parameters corresponding to three of the six parameters $X_1$, $X_2$, $X_3$, $X_4$, $X_9$, and $X_{10}$) is formulated.

$$\begin{aligned} C &= f(X_1, X_2, X_3) \\ &= a_0 + a_1 X_1 + a_2 X_2 + a_3 X_3 \quad \text{......(8)} \end{aligned}$$

Then, the least-squares method is employed to obtain a multiple regression equation that would minimize the error with respect to a measured value $C_i$ of glucose concentration according to an enzyme electrode method. When the sum of squares of the residual is D, D is expressed by the following equation (9):

$$\begin{aligned} D &= \sum_{i=1}^{n} {d_i}^2 \\ &= \sum_{i=1}^{n} (C_i - f(X_{i1}, X_{i2}, X_{i3}))^2 \\ &= \sum_{i=1}^{n} \{C_i - (a_0 + a_1 X_{i1} + a_2 X_{i2} + a_3 X_{i3})\}^2 \quad \text{......(9)} \end{aligned}$$

The sum of squares of the residual D becomes minimum when partial differentiation of equation (9) with respect to $a_0$ to $a_3$ gives zero. Thus, we have the following equations:

$$\frac{\partial D}{\partial a_0} = -2\sum_{i=1}^{n} \{C_i - (a_0 + a_1 X_{i1} + a_2 X_{i2} + a_3 X_{i3})\} = 0$$

$$\frac{\partial D}{\partial a_1} = -2\sum_{i=1}^{n} X_{i1}\{C_i - (a_0 + a_1X_{i1} + a_2X_{i2} + a_3X_{i3})\} = 0$$

$$\frac{\partial D}{\partial a_2} = -2\sum_{i=1}^{n} X_{i2}\{C_i - (a_0 + a_1X_{i1} + a_2X_{i2} + a_3X_{i3})\} = 0$$

$$\frac{\partial D}{\partial a_3} = -2\sum_{i=1}^{n} X_{i3}\{C_i - (a_0 + a_1X_{i1} + a_2X_{i2} + a_3X_{i3})\} = 0 \quad ......(10)$$

When the mean values of C and $X_1$ to $X_3$ are $C_{mean}$ and $X_{1mean}$ to $X_{3mean}$, respectively, since $X_{imean}=0$ (i=1 to 3), equation (8) yields equation (11) thus:

$$\begin{aligned} a_0 &= C_{mean} - a_1X_{1mean} - a_2X_{2mean} - a_3X_{3mean} \\ &= C_{mean} \end{aligned} \quad ......(11)$$

The variation and covariation between the normalized parameters are expressed by equation (12). Covariation between the normalized parameter $X_i$ (i=1 to 3) and C is expressed by equation (13).

$$S_{ij.} = \sum_{k=1}^{n}(X_{ki} - X_{imean})(X_{kj} - X_{jmean}) = \sum_{k=1}^{n} X_{ki}X_{kj} \quad (i, j = 1,2,3) \quad ......(12)$$

$$S_{iC} = \sum_{k=1}^{n}(X_{ki} - X_{imean})(C_k - C_{mean}) = \sum_{k=1}^{n} X_{ki}(C_k - C_{mean}) \quad (i = 1,2,3) \quad ......(13)$$

Substituting equations (11), (12), and (13) into equation (10) and rearranging yields simultaneous equations (normalized equations) (14). Solving equations (14) yields $a_1$ to $a_3$.

$$a_1S_{11} + a_2S_{12} + a_3S_{13} = S_{1C}$$

$$a_1S_{21} + a_2S_{22} + a_3S_{23} = S_{2C}$$

$$a_1S_{31} + a_2S_{32} + a_3S_{33} = S_{3C} \quad (14)$$

Constant term $a_0$ is obtained by means of equation (11). The thus obtained $a_i$ (i=0, 1, 2, 3) is stored in ROM at the time of manufacture of the apparatus. In actual measurement using the apparatus, the normalized parameters $X_1$ to $X_3$ obtained from the measured values are substituted into regression equation (8) to calculate the glucose concentration C.

Hereafter, an example of the process of calculating the glucose concentration will be described. The coefficients in equation (8) are determined in advance based on a large quantity of data obtained from able-bodied persons and diabetic patients. The ROM in the microprocessor stores the following formula for the calculation of glucose concentration. This formula can be obtained by cluster analysis, as described above. The coefficients of the formula and the average value and standard deviations of parameters $X_1$ to $X_3$ are updated to the latest data by communications with the server or the data storage machine.

$$C = 101.7+25.8\times X_1-23.2\times X_2-12.9\times X_3$$

$X_1$ to $X_3$ are the results of normalization of parameters $x_1$ to $x_3$. Assuming the distribution of the parameters is normal, 95% of the normalized parameters take on values between -2 and +2.

In an example of measured values for an able-bodied person, substituting normalized parameters $X_1$=-0.06, $X_2$=+0.04 and $X_3$=+0.10 in the above equation yields C=101 mg/dL. In an example of measured values for a diabetic patient, substituting normalized parameters $X_1$=+1.35, $X_2$=-1.22 and $X_3$=-1.24 in the equation yields C=181 mg/dL. In the above equation, the hemoglobin concentration and hemoglobin oxygen saturation are rendered into constants of 15 g/dL and 97%, respectively.

Hereafter, the results of measurement by the conventional enzymatic electrode method and those by the embodiment of the invention will be described. In the enzymatic electrode method, a blood sample is reacted with a reagent and the amount of resultant electrons is measured to determine glucose concentration. When the glucose concentration was 93 mg/dL according to the enzymatic electrode method in an example of measured values for an able-bodied person, substituting normalized parameters $X_1$=-0.06, $X_2$=+0.04 and $X_3$=+0.10 obtained by measurement at the same time according to the inventive method into the above equation yielded C=101 mg/dL. Further, when the glucose concentration was 208 mg/dL according to the enzymatic electrode method in an example of measurement values for a diabetic patient, substituting $X_1$=+1.35, $X_2$=-1.22 and $X_3$=-1.24 obtained by measurement at the same time according to the inventive method yielded C=181 mg/dL. Although the calculation results indicate an error of about 13%, this level of accuracy is considered sufficient because normally errors between 15% and 20% are considered acceptable in blood sugar level measuring apparatuses in general. Thus, it has been confirmed that the method of the invention can allow glucose concentrations to be determined with high accuracy.

Fig. 17 shows a chart plotting on the vertical axis the values of glucose concentration calculated by the inventive method and on the horizontal axis the values of glucose concentration measured by the enzymatic electrode method, based on measurement values obtained from a plurality of patients. A good correlation is obtained by measuring according to the invention (correlation coefficient = 0.8932).

## Claims

**1.** A blood sugar level calculation method comprising the steps of:

measuring a first temperature which is the temperature of a plate that is in contact with a body surface;
measuring a second temperature which is the temperature of the heat that is transmitted from said plate to a first member disposed adjacent to said plate;
measuring a third temperature which is the temperature of the heat radiating from said body surface;
detecting light with which said plate has been irradiated;
selecting a first calculation equation from a group of calculation equations on the basis of said first temperature, said second temperature, said third temperature, and the result of detection of said light, said group of calculation equations being obtained by classifying a plurality of data sets concerning said first temperature, said second temperature, said third temperature, and the result of detection of said light by a statistical process; and
calculating a blood sugar level by using said first temperature, said second temperature, said third temperature, said result of detection of light, and said first calculation equation.

**2.** The blood sugar level calculation method according to claim 1, wherein said first calculation equation is selected based on a calculation equation that relates said first temperature, said second temperature, said third temperature, and said result of detection of light to a blood sugar level.

**3.** A blood sugar level calculation method comprising the steps of:

measuring a first temperature which is the temperature of a plate that is in contact with a body surface;
measuring a second temperature which is the temperature of the heat that is transmitted from said plate to a columnar member disposed adjacent to said plate;
measuring a third temperature which is the temperature of the heat radiating from said body surface;
detecting light with which said plate has been irradiated;

selecting a first calculation equation from a group of calculation equations on the basis of said first temperature, said second temperature, said third temperature, and the result of detection of said light, said group of calculation equations being obtained by classifying a plurality of data sets concerning said first temperature, said second temperature, said third temperature, and the result of detection of said light by a statistical process; and calculating a blood sugar level by using said first temperature, said second temperature, said third temperature, said result of detection of light, and said first calculation equation.

**4.** The blood sugar level calculation method according to claim 3, wherein said first calculation equation is selected based on a calculation equation that relates said first temperature, said second temperature, said third temperature, and said result of detection of light to a blood sugar level.

**5.** A blood sugar level measuring system comprising:

a heat amount measuring portion for measuring a plurality of temperatures derived from a body surface and obtaining information used for calculating the amount of heat transferred by convection and the amount of heat transferred by radiation, both related to heat dissipation from said body surface;
a body surface contact portion;
an indirect temperature detector for detecting the concentration at a position spaced apart from said body surface contact portion;
a heat conducting member connecting said body surface contact portion and said indirect temperature detector;
a blood flow volume measuring portion for obtaining information relating to the volume of blood flow;
an optical measuring portion for obtaining hemoglobin concentration and hemoglobin oxygen saturation in blood;
an oxygen supply volume measuring portion for obtaining information about the amount of oxygen in blood;
a first storage portion for storing groups of measurement value sets or a calculation equation prepared for each of said groups of measurement value sets, said groups of measurement value sets being obtained by classifying a plurality of sets of measurement values obtained in advance by said heat amount measuring portion and said oxygen supply volume measuring portion by a statistical process;
a calculation equation selecting means for selecting a first calculation equation from said first storage portion based on measurement information about said plurality of temperatures and the blood oxygen amount;
a second storage portion for storing said first calculation equation;
a calculation portion for calculating a blood sugar level by using a plurality of measurement values inputted from said heat amount measuring portion and said oxygen supply volume measuring portion, and said first calculation equation stored in said second storage portion; and
a display portion for displaying the result of calculation in said calculation portion.

**6.** A blood sugar level measuring apparatus comprising:

an ambient temperature measuring device for measuring ambient temperature;
a body-surface contact portion to which a body surface is brought into contact;
a radiant heat detector for measuring radiant heat from said body surface;
a heat conducting member disposed in contact with said body-surface contact portion;
an indirect temperature detector disposed at a position that is adjacent to said heat conducting member and that is spaced apart from said body-surface contact portion, said indirect temperature detector measuring temperature at the position spaced apart from said body-surface contact portion;
a light source for irradiating said body-surface contact portion with light of at least two different wavelengths;
a light detector for detecting reflected light produced as said light is reflected by said body surface;
a first storage portion for storing groups of measurement value sets or a calculation equation prepared for each of said groups of measurement value sets, said groups of measurement value sets being obtained by classifying a plurality of sets of measurement values obtained in advance by said heat amount measuring portion and said oxygen supply volume measuring portion by a statistical process;
a calculation equation selecting means for selecting a first calculation equation from said first storage portion based on the outputs from said indirect temperature detector, said ambient temperature detector, said radiant heat detector, and said light detector;
a second storage portion for storing said first calculation equation;
a calculation portion for calculating a blood sugar level by using the outputs from said indirect temperature detector, said ambient temperature detector, said radiant heat detector, and said light detector, and said first calculation equation stored in said second storage portion; and
a display portion for displaying the blood sugar level outputted from said calculating portion.

**7.** The blood sugar level measuring system according to claim 6, further comprising:

an adjacent temperature detector for detecting the temperature of a plate covering an opening end of said heat conducting member adjacent to said body surface contact portion, and the temperature of said plate, wherein:

said sets include an output from said adjacent temperature detector, and
said calculation equation selecting means selects said first calculation equation from said first storage portion based on the outputs from said indirect temperature detector, said ambient temperature detector, said radiant heat detector, said light detector, and said adjacent temperature detector.

**8.** A blood sugar level measuring apparatus comprising:

an ambient temperature measuring device for measuring ambient temperature;
a body-surface contact portion to which a body surface is brought into contact;
a radiant heat detector for measuring radiant heat from said body surface;
a heat conducting member disposed in contact with said body-surface contact portion;
an indirect temperature detector disposed at a position that is adjacent to said heat conducting member and that is spaced apart from said body-surface contact portion, said indirect temperature detector measuring temperature at the position spaced apart from said body-surface contact portion;
a first storage portion storing information about hemoglobin concentration and hemoglobin oxygen saturation in blood;
a first storage portion for storing groups of measurement value sets or a calculation equation prepared for each of said groups of measurement value sets, said groups of measurement value sets being obtained by classifying a plurality of sets of measurement values obtained in advance by said heat amount measuring portion and said oxygen supply volume measuring portion by a statistical process;
a second storage portion for storing said first calculation equation;
a calculation portion for calculating a blood sugar level by using the outputs from said indirect temperature detector, said ambient temperature detector, said radiant heat detector, and said first calculation equation stored in said second storage portion; and
a display portion for displaying the blood sugar level outputted from said calculating portion.

**9.** The blood sugar level measuring system according to claim 8, further comprising:

an adjacent temperature detector for detecting the temperature of a plate covering an opening end of said heat conducting member adjacent to said body surface contact portion, and the temperature of said plate, wherein:

said sets include an output from said adjacent temperature detector, and
said calculation equation selecting means selects said first calculation equation from said first storage portion based on the outputs from said indirect temperature detector, said ambient temperature detector, said radiant heat detector, said light detector, and said adjacent temperature detector.

**10.** A blood sugar level measuring apparatus comprising:

a heat amount measurement portion for measuring a plurality of temperatures derived from a body surface and obtaining information used for calculating the amount of heat transferred by convection and the amount of heat transferred by radiation, both related to the dissipation of heat from said body surface;
an oxygen amount measuring portion for obtaining information about blood oxygen level;
a storage portion for storing a relationship between parameters corresponding to said plurality of temperatures and blood oxygen amount and blood sugar levels;
a calculating portion which converts a plurality of measurement values fed from said heat amount measuring portion and said oxygen amount measurement portion into said parameters, and computes a blood sugar level by applying said parameters to said relationship stored in said storage portion;
a display portion for displaying the blood sugar level calculated by said calculating portion;

a communication interface; and
a control portion for replacing said relationship stored in said storage portion with said relationship acquired from said communication interface, wherein:

said oxygen level measurement portion includes a blood flow volume measurement portion for obtaining information about blood flow volume, and an optical measurement portion for obtaining blood hemoglobin concentration and hemoglobin oxygen saturation, wherein said blood flow volume measurement portion includes:

a body-surface contact portion;
an indirect temperature detector for detecting the concentration at a position spaced apart from said body-surface contact portion; and
a heat conducting member connecting said body-surface contact portion and said indirect temperature detector.

**11.** The blood sugar level measuring apparatus according to claim 10, wherein said storage portion stores an average value and standard deviation value of each of said parameters, and wherein said control portion replaces the average value and standard deviation value of each parameter stored in said storage portion with an average value and standard deviation value acquired via said communication interface.

**12.** A blood sugar level measuring apparatus comprising:

an ambient temperature measuring device for measuring ambient temperature;
a body-surface contact portion to which a body surface is brought into contact;
a radiant heat detector for measuring radiant heat from said body surface;
a heat conducting member disposed in contact with said body-surface contact portion;
an indirect temperature detector disposed at a position that is adjacent to said heat conducting member and that is spaced apart from said body-surface contact portion, said indirect temperature detector measuring temperature at the position spaced apart from said body-surface contact portion;
a light source for irradiating said body-surface contact portion with light of at least two different wavelengths;
a light detector for detecting reflected light produced as said light is reflected by said body surface;
a converter for converting outputs from said indirect temperature detector, said ambient temperature detector, said radiant heat detector and said light detector, into parameters;
a calculating portion in which a relationship between said parameters and blood sugar levels is stored in advance, and which calculates a blood sugar level by applying said parameters to said relationship;
a display portion for displaying the blood sugar level outputted from said calculating portion;
a communication interface; and
a control portion for replacing said relationship stored in said storage portion with said relationship acquired via said communication interface.

**13.** The blood sugar level measuring apparatus according to claim 12, wherein said storage portion stores an average value and standard deviation value of each of said parameters, and wherein said control portion replaces the average value and standard deviation value of each parameter stored in said storage portion with an average value and standard deviation value acquired via said communication interface.

**14.** A blood sugar level measuring apparatus comprising:

an ambient temperature measuring device for measuring ambient temperature;
a body-surface contact portion to which a body surface is brought into contact;
a radiant heat detector for measuring radiant heat from said body surface;
a heat conducting member disposed in contact with said body-surface contact portion;
an indirect temperature detector disposed at a position that is adjacent to said heat conducting member and that is spaced apart from said body-surface contact portion, said indirect temperature detector measuring temperature at the position spaced apart from said body-surface contact portion;
a storage portion storing information about hemoglobin concentration and hemoglobin oxygen saturation in blood;
a converter for converting outputs from said indirect temperature detector, said ambient temperature detector, said radiant heat detector into a plurality of parameters;

a calculating portion in which a relationship between said parameters and blood sugar levels is stored in advance, and which calculates a blood sugar level by applying said parameters to said relationship;
a display portion for displaying the blood sugar level outputted from said calculating portion;
a communication interface; and
a control portion for replacing said relationship stored in said storage portion with said relationship acquired via said communication interface.

**15.** The blood sugar level measuring apparatus according to claim 14, wherein said storage portion stores an average value and standard deviation value of each of said parameters, and wherein said control portion replaces the average value and standard deviation value of each parameter stored in said storage portion with an average value and standard deviation value acquired via said communication interface.

**16.** A system comprising:

a receiver portion adapted to receive a measurement data set including the temperature of a body surface, the temperature of a heat conducting member that is in contact with said body surface, the radiation temperature on said body surface, the values of a plurality of parameters calculated from ambient temperature, and a blood sugar level measured by a usual method;
a measurement data storage portion storing a plurality of measurement data sets received by said receiver portion;
a function data storage portion storing an average value and standard deviation value of each parameter, and a relation expression indicating the relationship between said parameters and blood sugar levels;
a processing portion for statistically processing the multiple data sets stored in said measurement data storage portion, determining a relation expression indicating the relationship between said parameters and blood sugar levels, and storing the relation expression in said function data storage portion; and
a transmitter portion for transmitting the average value and standard deviation value of each parameter stored in said function data storage portion and the relation expression indicating the relationship between said parameters and blood sugar levels.

**17.** The system according to claim 16, wherein said multiple parameters are calculated based on absorbance at at least two wavelengths of the light with which said body surface is irradiated, in addition to the temperature of the body surface, the temperature of the heat conducting member in contact with said body surface, the radiant temperature of said body surface, and the ambient temperature.

**18.** The system according to claim 16, wherein said processing portion re-calculates the relation expression indicating the average value and standard deviation value of each parameter and the relationship between the parameters and blood sugar levels when the number of the measurement data sets additionally stored in said measurement data storage portion, or the number of data items stored in said measurement data storage portion has reached a predetermined number, and updates the data stored in said function data storage portion.

FIG. 1

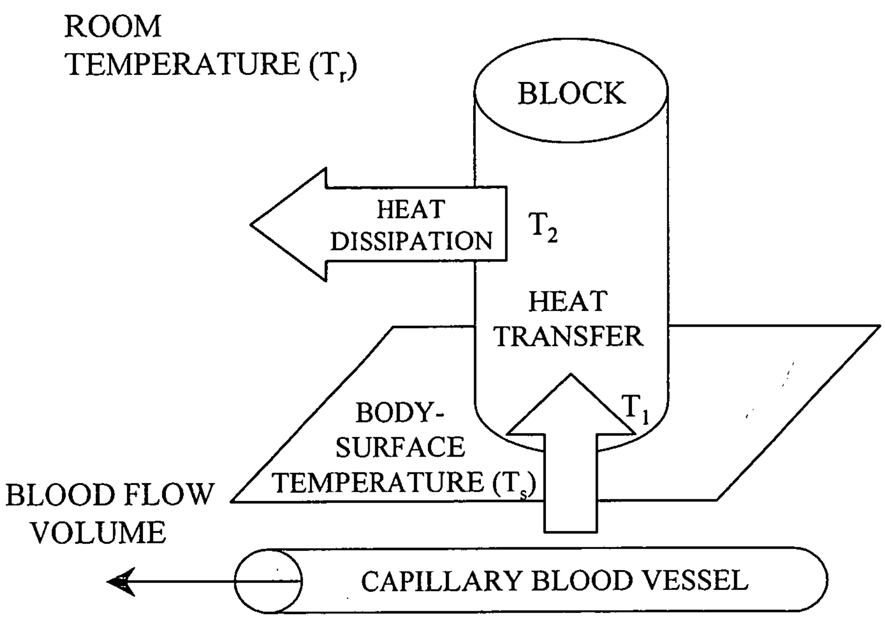
ROOM
TEMPERATURE ($T_r$)
BLOCK
HEAT
DISSIPATION
$T_2$
HEAT
TRANSFER
BODY-
SURFACE
TEMPERATURE ($T_s$)
$T_1$
BLOOD FLOW
VOLUME
CAPILLARY BLOOD VESSEL

FIG. 2

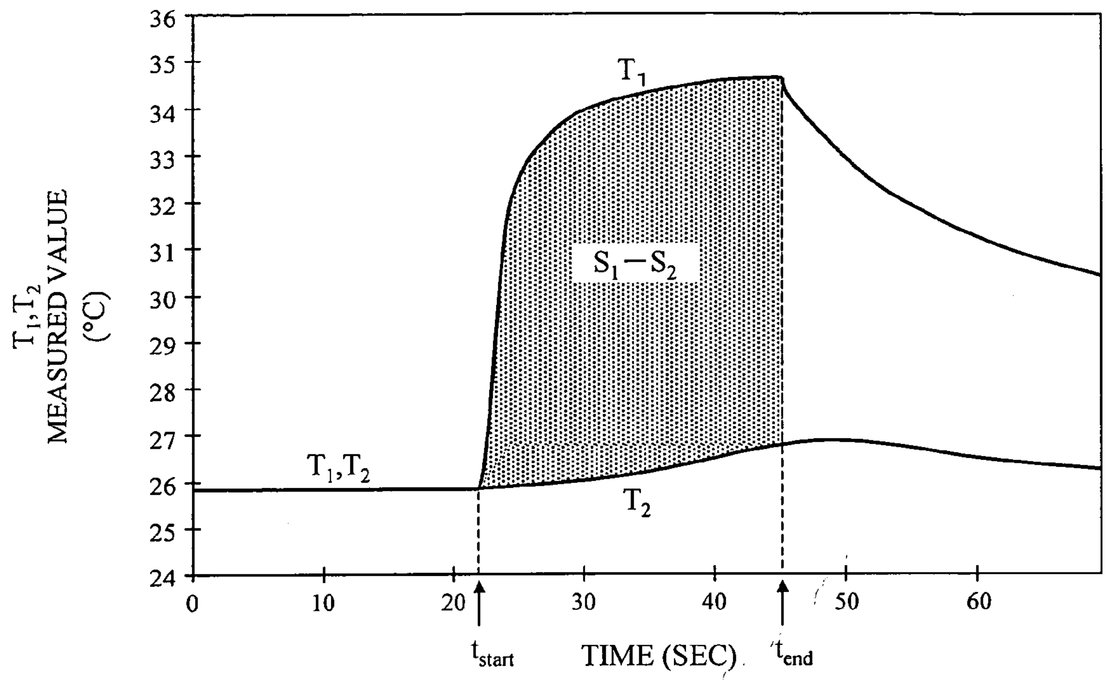
36
35
34
33
32
31
30
29
28
27
26
25
24
T1,T2
MEASURED VALUE
(°C)
T1
S1−S2
T1,T2
T2
0
10
20
30
40
50
60
tstart
TIME (SEC)
tend

FIG. 3

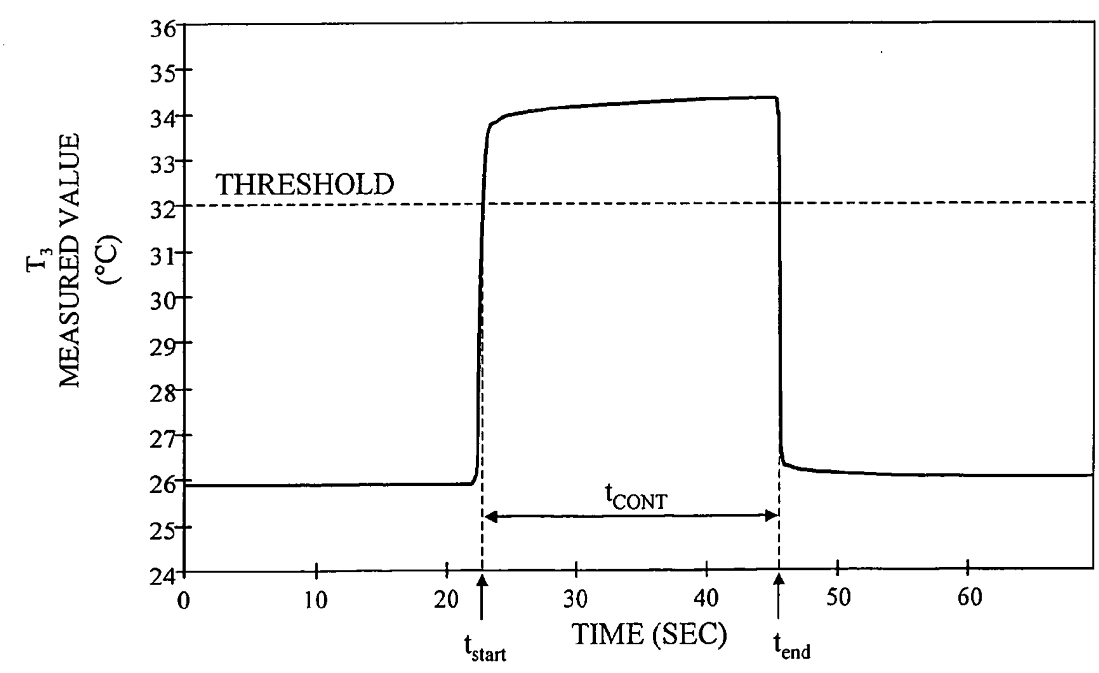
36
35
34
33
32
31
30
29
28
27
26
25
24
T3
MEASURED VALUE
(°C)
THRESHOLD
tCONT
0
10
20
30
40
50
60
tstart
TIME (SEC)
tend

FIG. 4

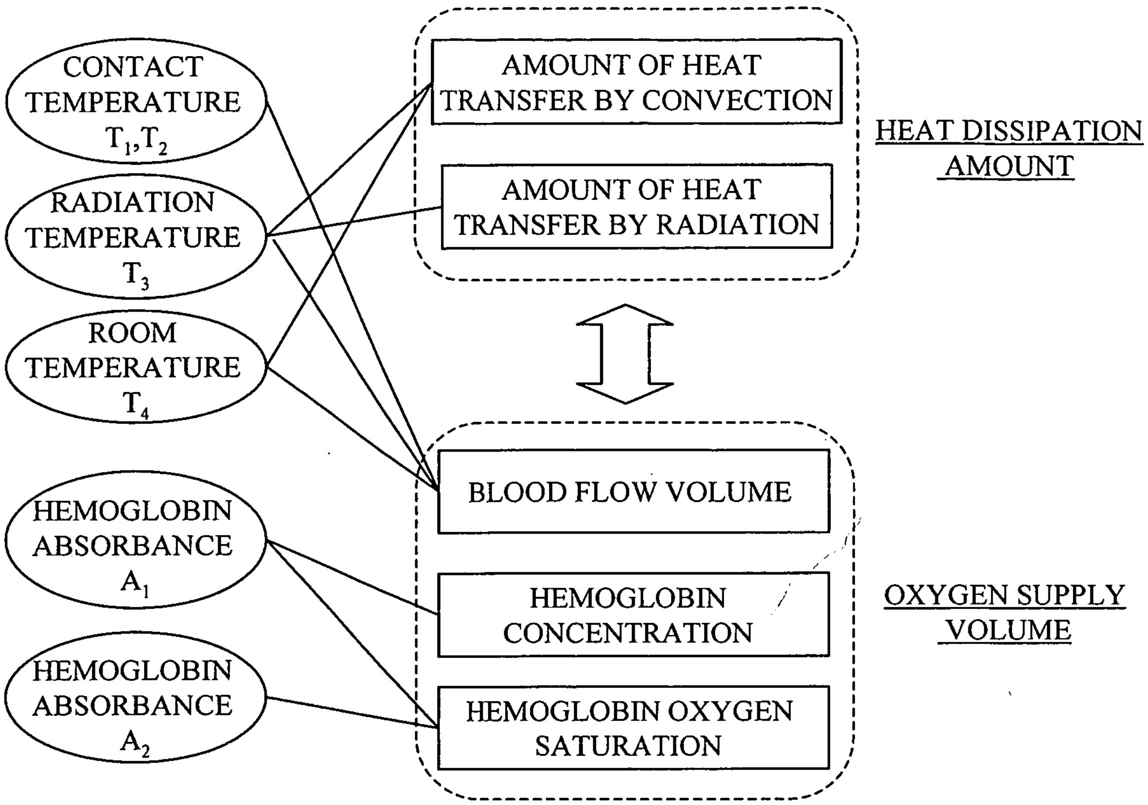
CONTACT TEMPERATURE $T_1$,$T_2$
RADIATION TEMPERATURE $T_3$
ROOM TEMPERATURE $T_4$
HEMOGLOBIN ABSORBANCE $A_1$
HEMOGLOBIN ABSORBANCE $A_2$
AMOUNT OF HEAT TRANSFER BY CONVECTION
AMOUNT OF HEAT TRANSFER BY RADIATION
HEAT DISSIPATION AMOUNT
BLOOD FLOW VOLUME
HEMOGLOBIN CONCENTRATION
HEMOGLOBIN OXYGEN SATURATION
OXYGEN SUPPLY VOLUME

FIG. 5

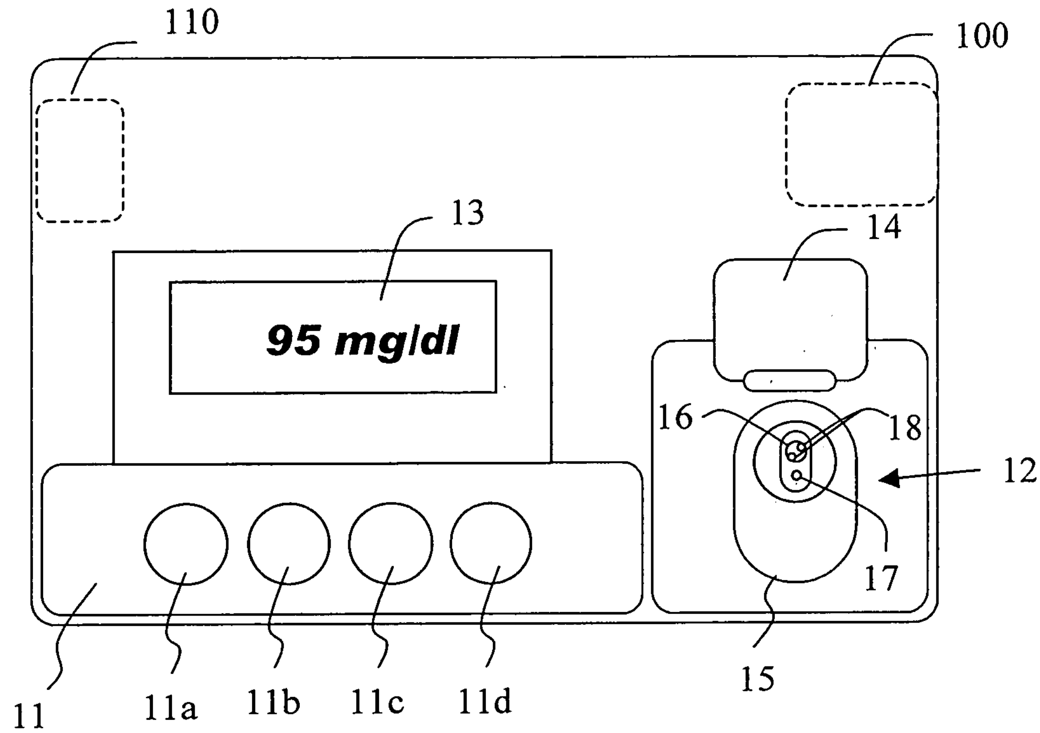

FIG. 6

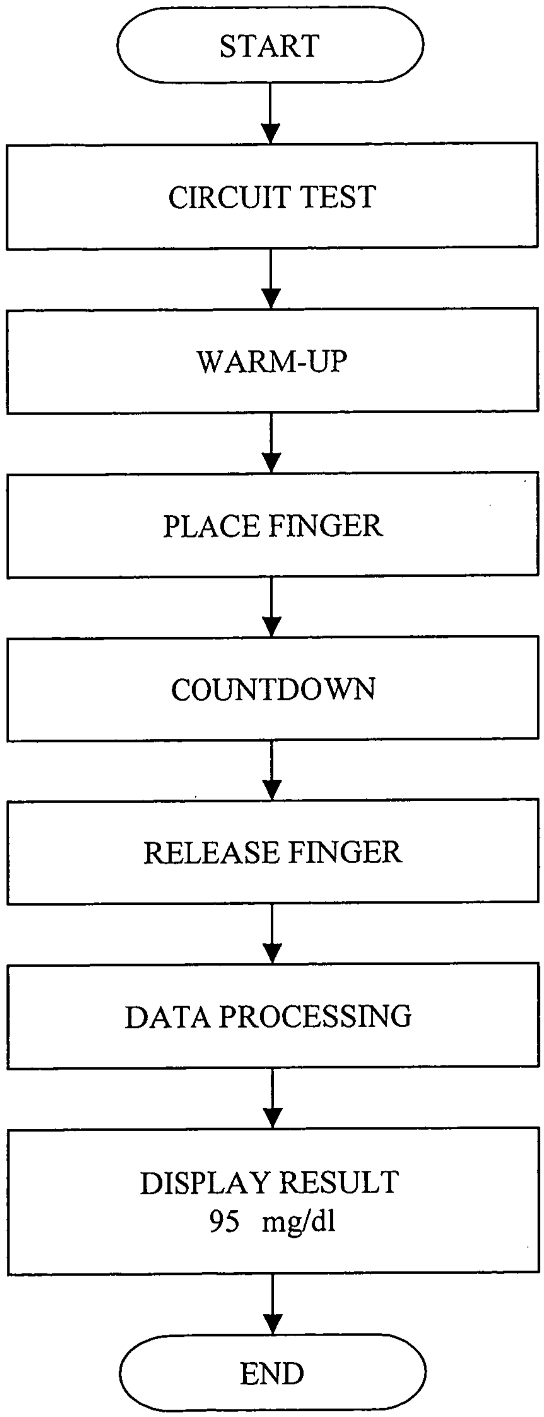
START
CIRCUIT TEST
WARM-UP
PLACE FINGER
COUNTDOWN
RELEASE FINGER
DATA PROCESSING
DISPLAY RESULT
95 mg/dl
END

FIG. 7

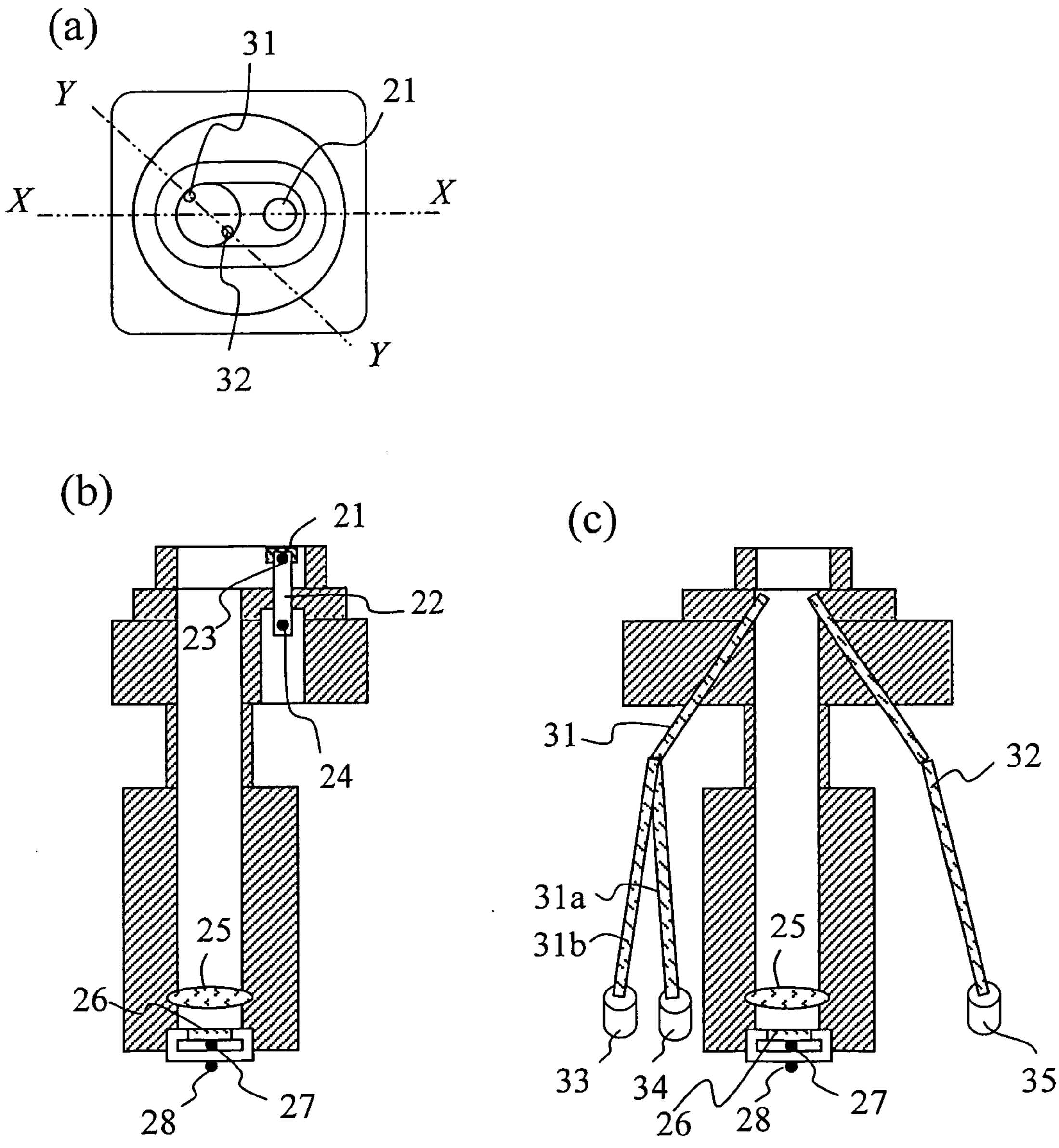
(a)
31
21
Y
X
X
Y
32
(b)
21
22
23
24
25
26
27
28
(c)
31
32
31a
31b
25
33
34
26
28
27
35

FIG. 8

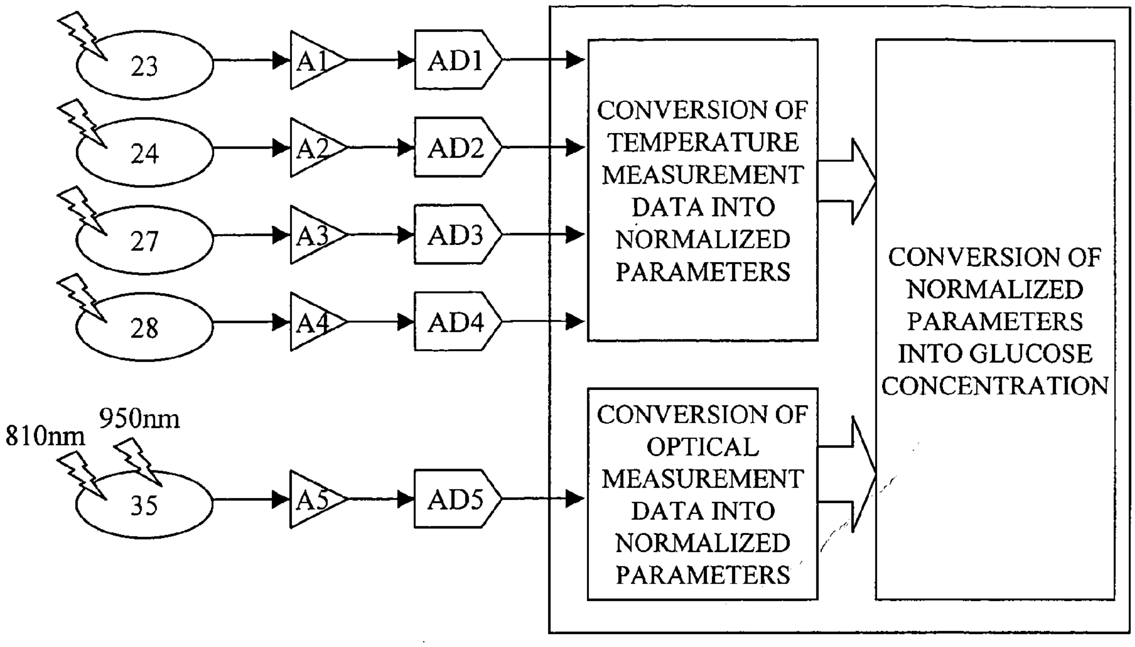
23
24
27
28
810nm
950nm
35
A1
A2
A3
A4
A5
AD1
AD2
AD3
AD4
AD5
CONVERSION OF TEMPERATURE MEASUREMENT DATA INTO NORMALIZED PARAMETERS
CONVERSION OF OPTICAL MEASUREMENT DATA INTO NORMALIZED PARAMETERS
CONVERSION OF NORMALIZED PARAMETERS INTO GLUCOSE CONCENTRATION

FIG. 9

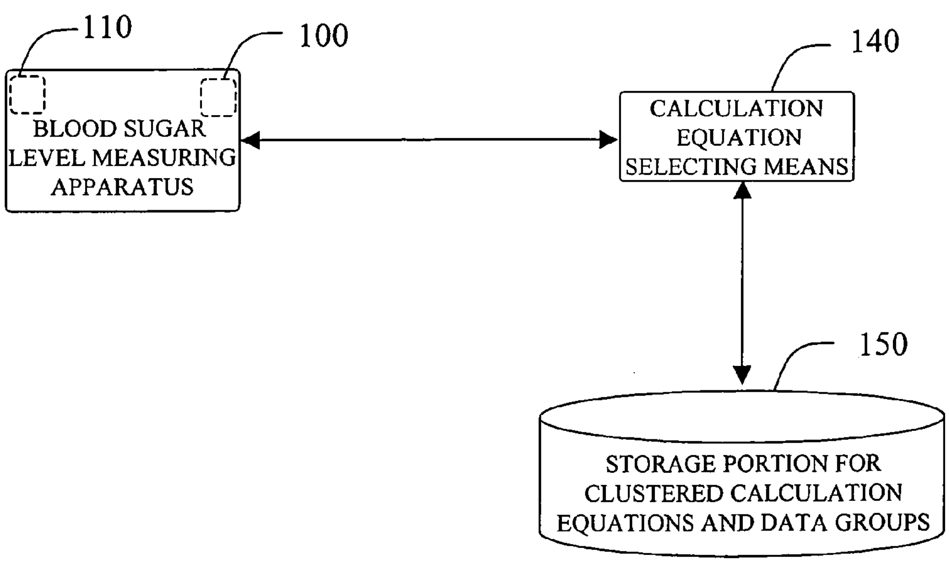
110
100
BLOOD SUGAR LEVEL MEASURING APPARATUS
140
CALCULATION EQUATION SELECTING MEANS
150
STORAGE PORTION FOR CLUSTERED CALCULATION EQUATIONS AND DATA GROUPS

FIG. 10

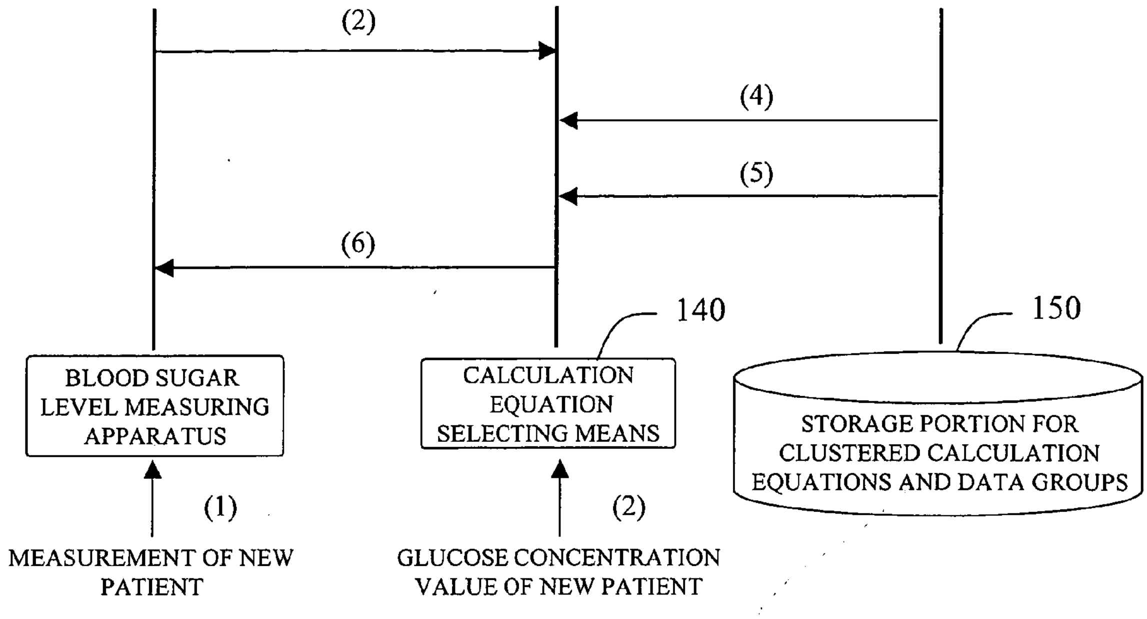
(2)
(4)
(5)
(6)
140
150
BLOOD SUGAR LEVEL MEASURING APPARATUS
CALCULATION EQUATION SELECTING MEANS
STORAGE PORTION FOR CLUSTERED CALCULATION EQUATIONS AND DATA GROUPS
(1)
(2)
MEASUREMENT OF NEW PATIENT
GLUCOSE CONCENTRATION VALUE OF NEW PATIENT

FIG. 11

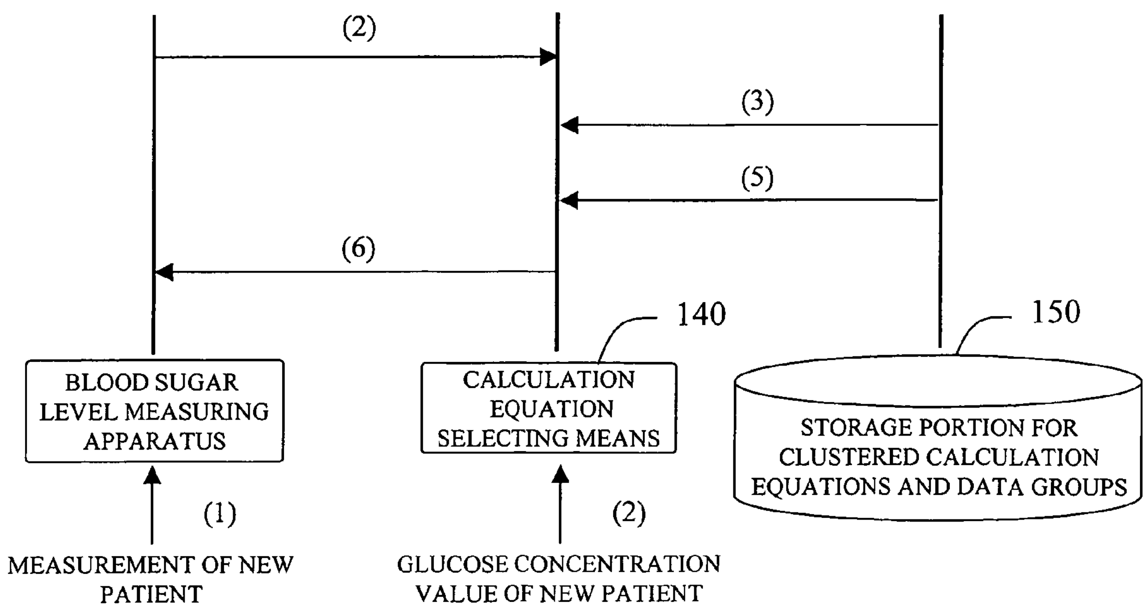
(2)
(3)
(5)
(6)
140
150
BLOOD SUGAR LEVEL MEASURING APPARATUS
CALCULATION EQUATION SELECTING MEANS
STORAGE PORTION FOR CLUSTERED CALCULATION EQUATIONS AND DATA GROUPS
(1)
(2)
MEASUREMENT OF NEW PATIENT
GLUCOSE CONCENTRATION VALUE OF NEW PATIENT

FIG. 12

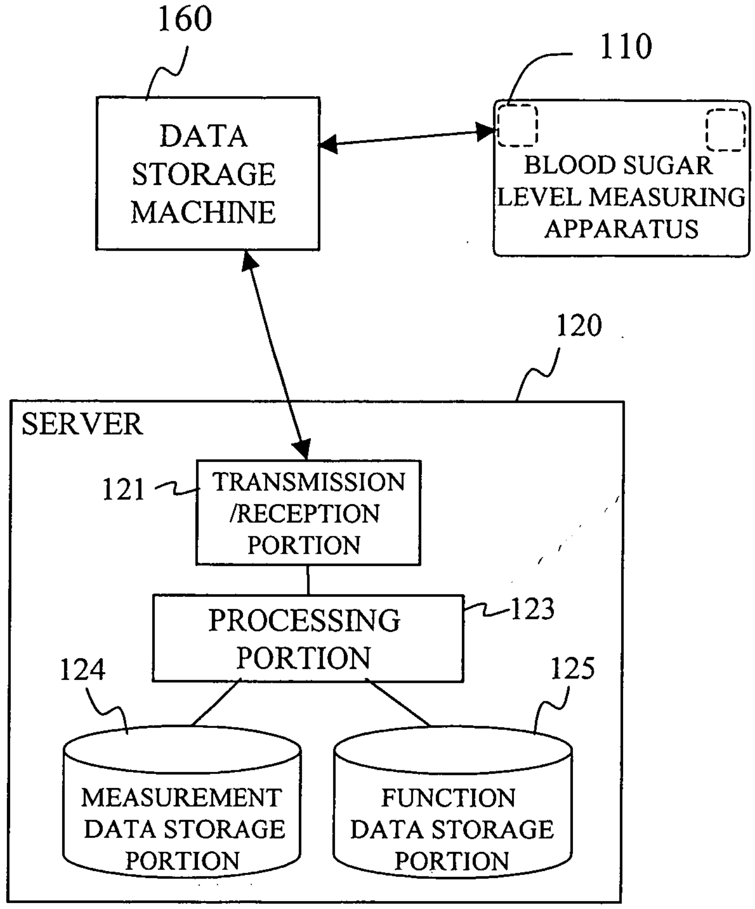
160
110
DATA STORAGE MACHINE
BLOOD SUGAR LEVEL MEASURING APPARATUS
120
SERVER
121
TRANSMISSION /RECEPTION PORTION
PROCESSING PORTION
123
124
125
MEASUREMENT DATA STORAGE PORTION
FUNCTION DATA STORAGE PORTION

FIG. 13

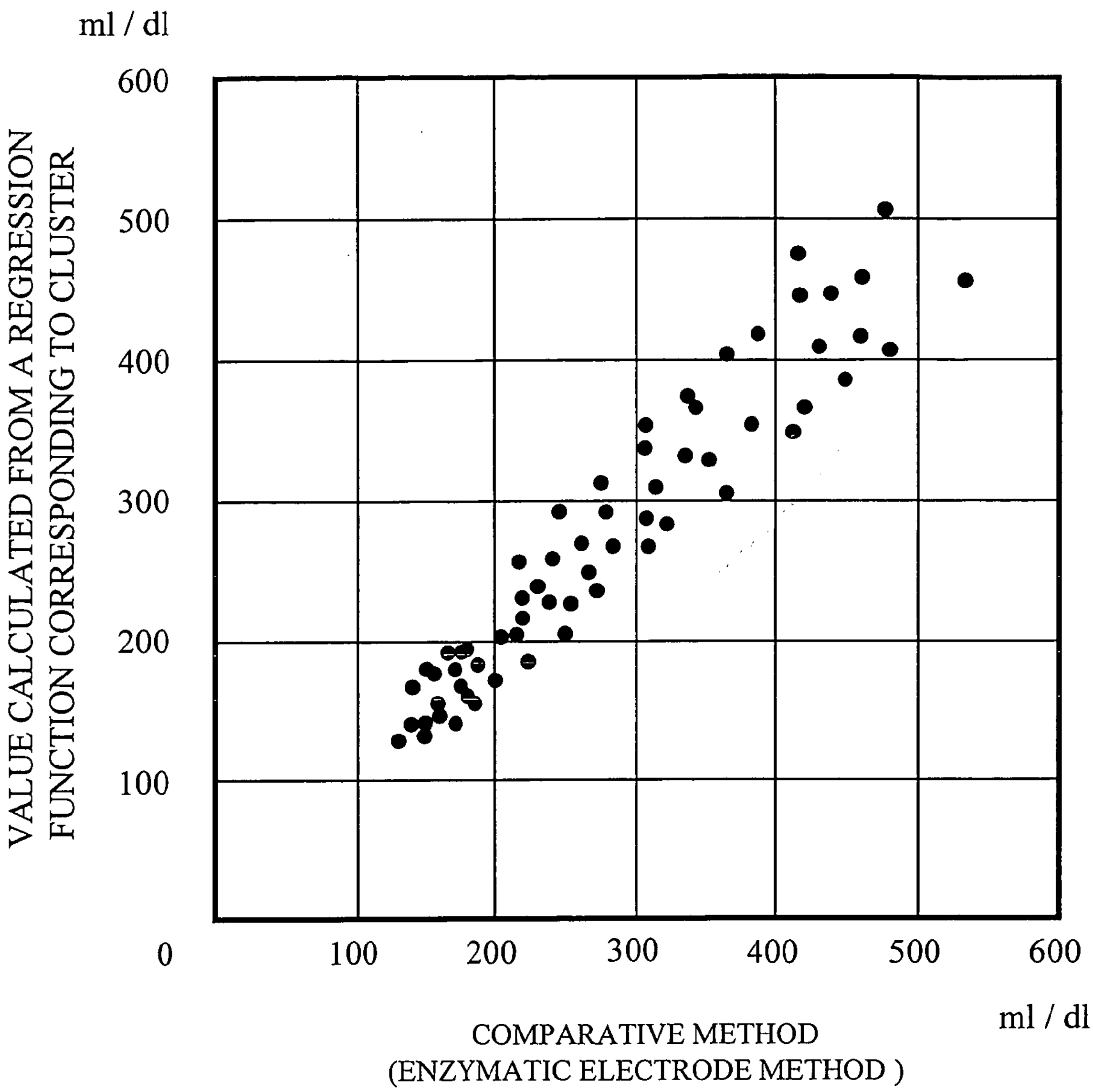
ml / dl
600
500
400
300
200
100
0
VALUE CALCULATED FROM A REGRESSION FUNCTION CORRESPONDING TO CLUSTER
100
200
300
400
500
600
ml / dl
COMPARATIVE METHOD (ENZYMATIC ELECTRODE METHOD )

FIG. 14

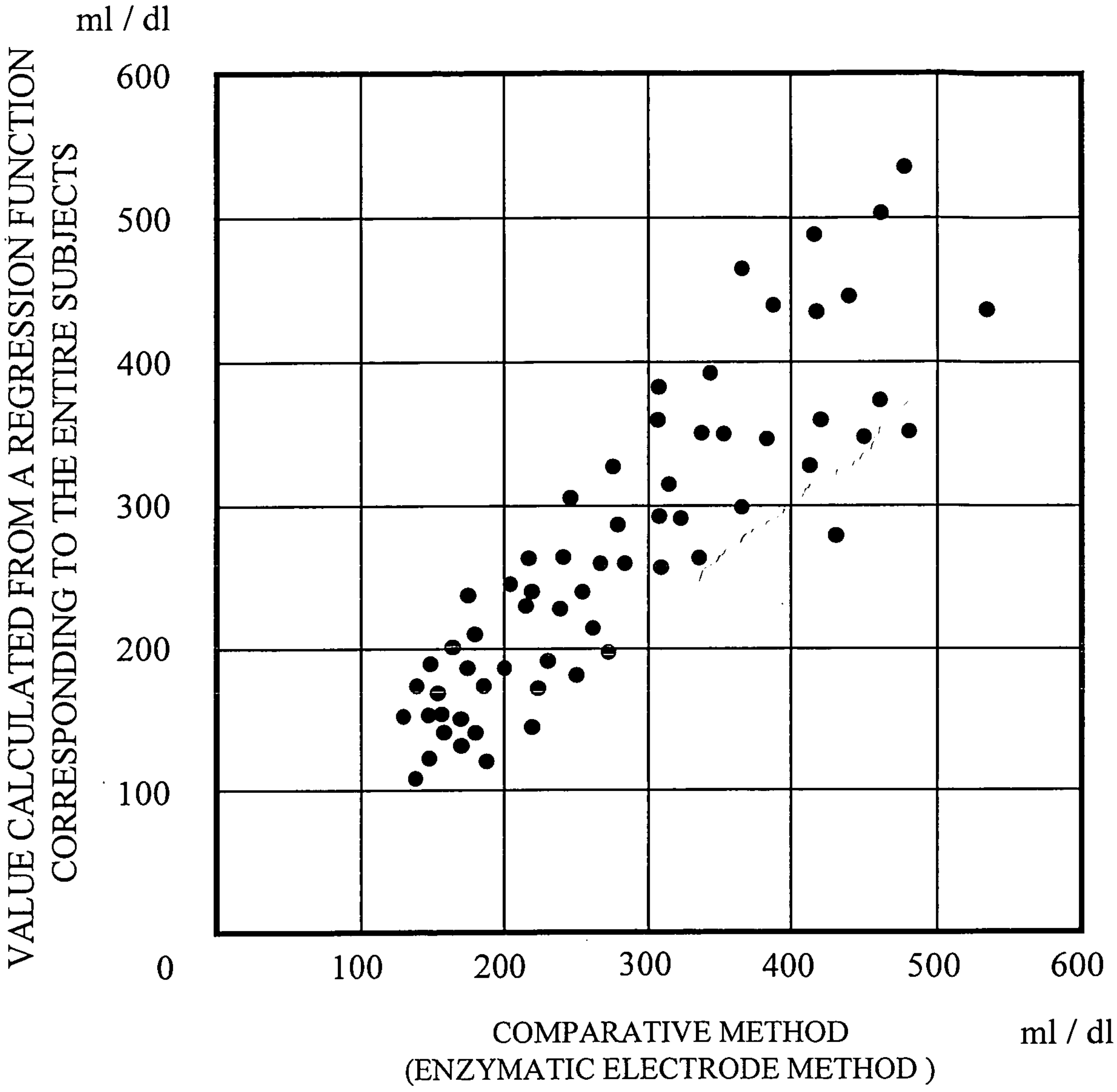
ml / dl
600
500
400
300
200
100
0
VALUE CALCULATED FROM A REGRESSION FUNCTION CORRESPONDING TO THE ENTIRE SUBJECTS
100
200
300
400
500
600
COMPARATIVE METHOD
(ENZYMATIC ELECTRODE METHOD )
ml / dl

FIG. 15

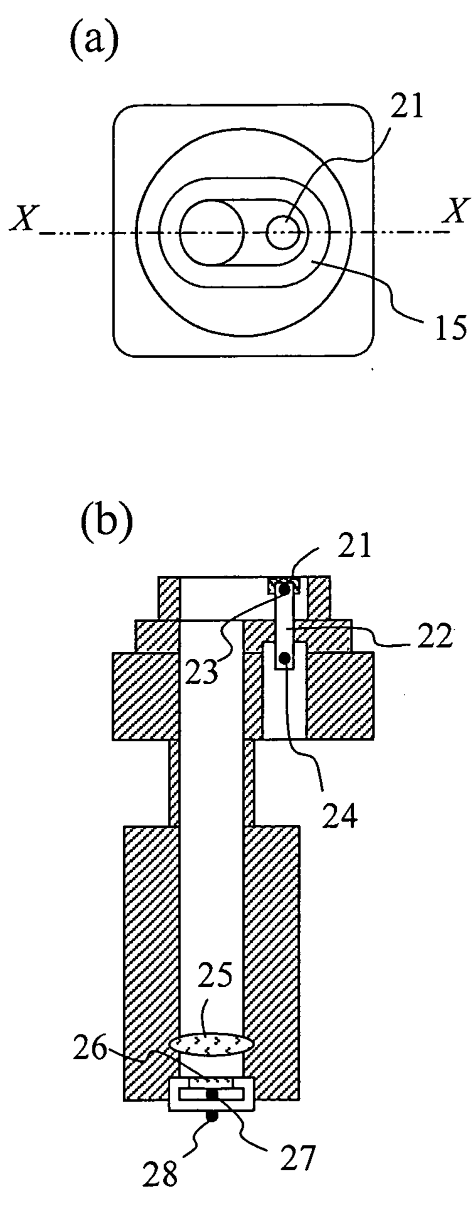

FIG. 16

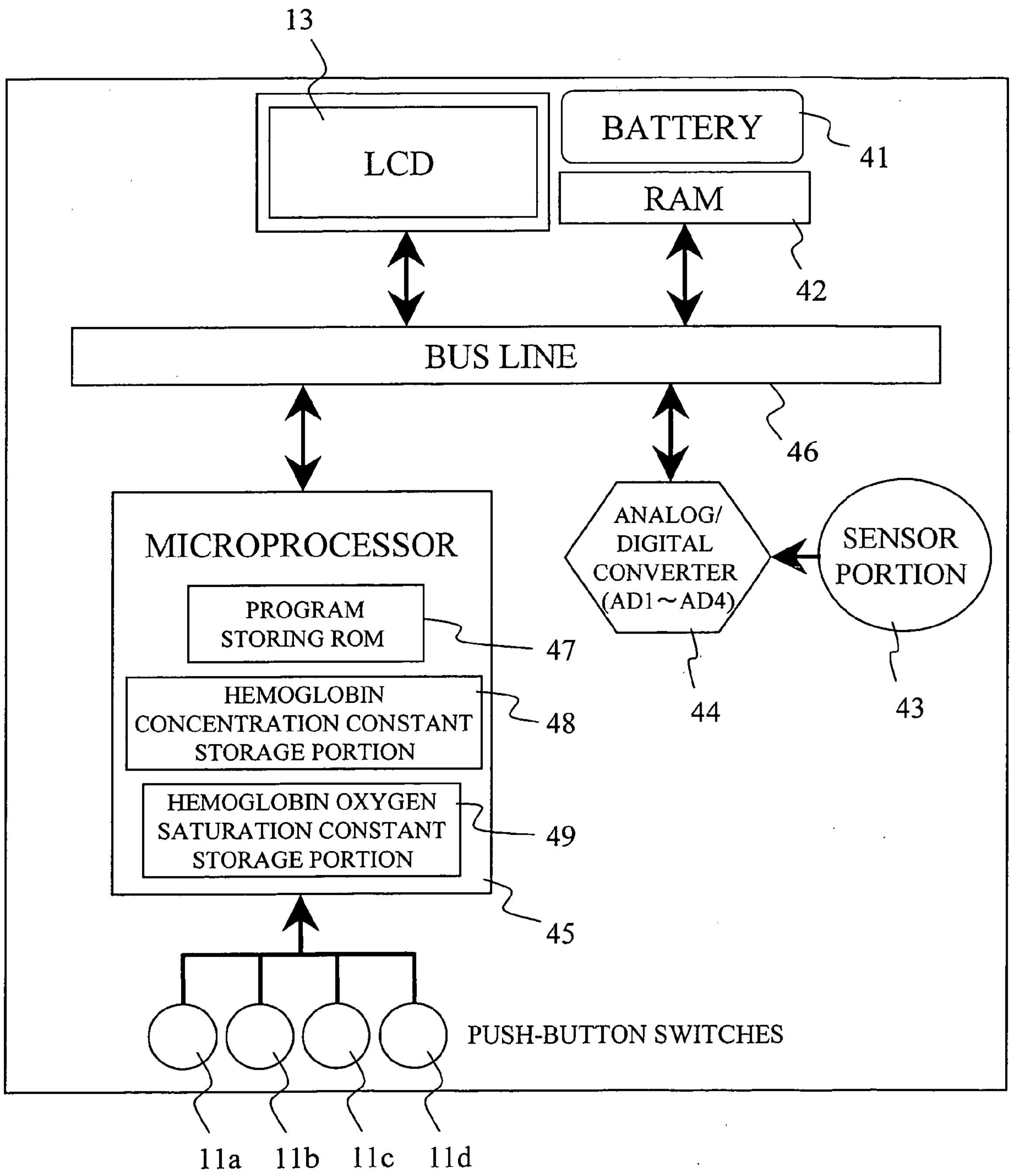
13
LCD
BATTERY
41
RAM
42
BUS LINE
46
MICROPROCESSOR
PROGRAM STORING ROM
47
HEMOGLOBIN CONCENTRATION CONSTANT STORAGE PORTION
48
HEMOGLOBIN OXYGEN SATURATION CONSTANT STORAGE PORTION
49
45
ANALOG/ DIGITAL CONVERTER (AD1~AD4)
44
SENSOR PORTION
43
PUSH-BUTTON SWITCHES
11a
11b
11c
11d

FIG. 17

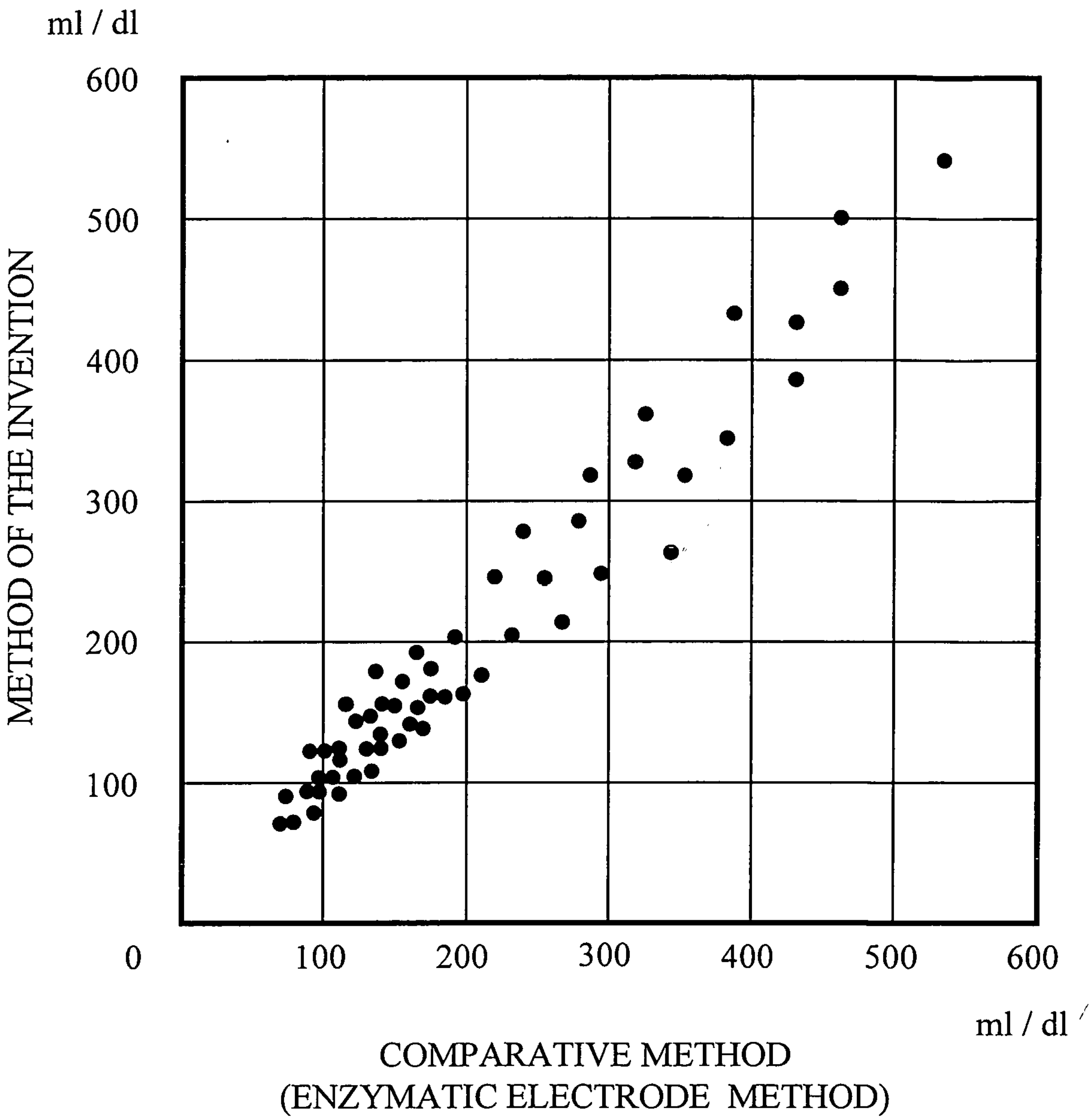
ml / dl
600
500
400
300
200
100
0
METHOD OF THE INVENTION
100
200
300
400
500
600
ml / dl
COMPARATIVE METHOD
(ENZYMATIC ELECTRODE METHOD)

European Patent Office

# EUROPEAN SEARCH REPORT

Application Number
EP 04 00 7818

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y<br><br><br><br>X | WO 01/28414 A (CHO OK KYUNG ; KAUFMANN KIM YUN OAK (DE)) 26 April 2001 (2001-04-26)<br>* page 1, lines 6-15 *<br>* page 7, lines 5-21 *<br>* page 13, line 1 - page 14, line 13 *<br>----- | 10,12<br><br><br><br>16,17 | A61B5/00<br>G01K13/00<br>G01K17/08<br>G06F17/00<br>G06F19/00 |
| D,Y | US 5 924 996 A (CHO ET AL)<br>20 July 1999 (1999-07-20)<br>* column 4, line 53 - column 5, line 44 *<br>* column 7, lines 21-27 *<br>* column 7, lines 32-65 *<br>----- | 10,12 | |
| A | US 5 576 544 A (ROSENTHAL ET AL)<br>19 November 1996 (1996-11-19)<br>* abstract *<br>* column 1, line 23 - column 2, line 62 *<br>* column 3, line 60 - column 4, line 62 *<br>----- | 1-8 | |
| A | WO 02/25233 A (INSTRUMENTATION METRICS, INC) 28 March 2002 (2002-03-28)<br>* page 10, lines 30-39 *<br>----- | 1 | TECHNICAL FIELDS SEARCHED (Int.Cl.7) |
| A | US 6 512 936 B1 (MONFRE STEPHEN L ET AL)<br>28 January 2003 (2003-01-28)<br>* column 11, lines 26-45 *<br>* column 5, line 37 - column 6, line 23 *<br>----- | 1 | A61B<br>G01K<br>G06F |
| E | EP 1 522 254 A (HITACHI, LTD)<br>13 April 2005 (2005-04-13)<br>* paragraphs [0046] - [0060]; figure 11 *<br>----- | 16,17 | |

The present search report has been drawn up for all claims

| Place of search | Date of completion of the search | Examiner |
|---|---|---|
| Berlin | 1 June 2005 | Kronberger, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

1

EPO FORM 1503 03.82 (P04C01)

## ANNEX TO THE EUROPEAN SEARCH REPORT ON EUROPEAN PATENT APPLICATION NO. EP 04 00 7818

This annex lists the patent family members relating to the patent documents cited in the above-mentioned European search report.
The members are as contained in the European Patent Office EDP file on
The European Patent Office is in no way liable for these particulars which are merely given for the purpose of information.

01-06-2005

| Patent document cited in search report | Publication date | Patent family member(s) | Publication date |
|---|---|---|---|
| WO 0128414 A | 26-04-2001 | WO 0128414 A2 | 26-04-2001 |
| US 5924996 A | 20-07-1999 | DE 4423663 A1 | 11-01-1996 |
| | | CA 2194348 A1 | 18-01-1996 |
| | | CN 1159151 A | 10-09-1997 |
| | | WO 9601075 A1 | 18-01-1996 |
| | | EP 0771168 A1 | 07-05-1997 |
| | | JP 10503944 T | 14-04-1998 |
| | | KR 271095 B1 | 01-12-2000 |
| US 5576544 A | 19-11-1996 | US 5204532 A | 20-04-1993 |
| | | US 5068536 A | 26-11-1991 |
| | | US 5077476 A | 31-12-1991 |
| | | US 5086229 A | 04-02-1992 |
| | | US 5028787 A | 02-07-1991 |
| | | US 6066847 A | 23-05-2000 |
| | | AU 2251292 A | 12-01-1993 |
| | | CA 2111868 A1 | 23-12-1992 |
| | | EP 0590077 A1 | 06-04-1994 |
| | | JP 6508440 T | 22-09-1994 |
| | | MX 9202953 A1 | 01-02-1993 |
| | | WO 9222804 A1 | 23-12-1992 |
| | | AT 145988 T | 15-12-1996 |
| | | AU 8238791 A | 23-01-1992 |
| | | CA 2086019 A1 | 28-12-1991 |
| | | DE 69123448 D1 | 16-01-1997 |
| | | DE 69123448 T2 | 22-05-1997 |
| | | EP 0536304 A1 | 14-04-1993 |
| | | IE 912231 A1 | 01-01-1992 |
| | | JP 5508336 T | 25-11-1993 |
| | | NZ 238717 A | 26-08-1994 |
| | | WO 9200513 A1 | 09-01-1992 |
| | | US 5362966 A | 08-11-1994 |
| | | US 5436455 A | 25-07-1995 |
| | | US 5438201 A | 01-08-1995 |
| | | US 5574283 A | 12-11-1996 |
| | | US 5237178 A | 17-08-1993 |
| | | US 5365066 A | 15-11-1994 |
| | | US 5218207 A | 08-06-1993 |
| | | ZA 9104977 A | 29-04-1992 |
| | | AT 163844 T | 15-03-1998 |
| | | AU 5042190 A | 13-08-1990 |
| | | CA 2045599 A1 | 20-07-1990 |
| | | DE 69032126 D1 | 16-04-1998 |
| | | DE 69032126 T2 | 15-10-1998 |
| | | EP 0456716 A1 | 21-11-1991 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 04 00 7818

This annex lists the patent family members relating to the patent documents cited in the above-mentioned European search report.
The members are as contained in the European Patent Office EDP file on
The European Patent Office is in no way liable for these particulars which are merely given for the purpose of information.

01-06-2005

| Patent document cited in search report | | Publication date | Patent family member(s) | | | Publication date |
|---|---|---|---|---|---|---|
| US 5576544 | A | | ES | 2114860 | T3 | 16-06-1998 |
| | | | JP | 2191434 | A | 27-07-1990 |
| | | | JP | 5058735 | B | 27-08-1993 |
| | | | KR | 145695 | B1 | 01-08-1998 |
| | | | WO | 9007905 | A1 | 26-07-1990 |
| WO 0225233 | A | 28-03-2002 | US | 6864978 | B1 | 08-03-2005 |
| | | | AU | 8341401 | A | 02-04-2002 |
| | | | CN | 1483141 | A | 17-03-2004 |
| | | | EP | 1319176 | A2 | 18-06-2003 |
| | | | EP | 1442699 | A1 | 04-08-2004 |
| | | | JP | 2004526938 | T | 02-09-2004 |
| | | | WO | 0225233 | A2 | 28-03-2002 |
| | | | US | 2004223155 | A1 | 11-11-2004 |
| US 6512936 | B1 | 28-01-2003 | US | 6280381 | B1 | 28-08-2001 |
| | | | AU | 8348001 | A | 02-04-2002 |
| | | | TW | 567056 | B | 21-12-2003 |
| | | | WO | 0223971 | A2 | 28-03-2002 |
| | | | AU | 754677 | B2 | 21-11-2002 |
| | | | AU | 2620900 | A | 07-08-2000 |
| | | | BR | 0008186 | A | 06-11-2001 |
| | | | CA | 2358473 | A1 | 27-07-2000 |
| | | | EP | 1143850 | A1 | 17-10-2001 |
| | | | JP | 2002535023 | T | 22-10-2002 |
| | | | NZ | 513092 | A | 28-02-2003 |
| | | | TW | 498156 | B | 11-08-2002 |
| | | | WO | 0042907 | A1 | 27-07-2000 |
| | | | US | 2003060693 | A1 | 27-03-2003 |
| | | | US | 2003023148 | A1 | 30-01-2003 |
| | | | US | 6501982 | B1 | 31-12-2002 |
| | | | US | 6405065 | B1 | 11-06-2002 |
| | | | US | 6493566 | B1 | 10-12-2002 |
| | | | US | 6587702 | B1 | 01-07-2003 |
| | | | US | 6475800 | B1 | 05-11-2002 |
| | | | US | 6456870 | B1 | 24-09-2002 |
| | | | US | 6864978 | B1 | 08-03-2005 |
| | | | US | 6442408 | B1 | 27-08-2002 |
| | | | US | 2001021803 | A1 | 13-09-2001 |
| | | | US | 2004223155 | A1 | 11-11-2004 |
| EP 1522254 | A | 13-04-2005 | JP | 3623498 | B1 | 23-02-2005 |
| | | | JP | 2005110995 | A | 28-04-2005 |
| | | | EP | 1522254 | A1 | 13-04-2005 |
| | | | US | 2005080324 | A1 | 14-04-2005 |

EPO FORM P0459

For more details about this annex : see Official Journal of the European Patent Office, No. 12/82